# EUROPEAN PATENT SPECIFICATION

(11) **EP 0 528 931 B1**
(45) Date of publication and mention of the grant of the patent: **08.07.1998**
(21) Application number: 91909612.3
(22) Date of filing: 29.04.1991
(51) Int. Cl.: A61K 39/395, C07K 16/46

(54) **HUMANIZED CHIMERIC ANTI-ICAM-1 ANTIBODIES, METHODS OF PREPARATION AND USE**
HUMANISIERTE CHIMÄRE ANTI-"ICAM-1" ANTIKÖRPER, HERSTELLUNGSVERFAHREN UND VERWENDUNG
ANTICORPS ANTI-MOLECULE 1 D'ADHERENCE INTERCELLULAIRE CHIMERIQUE ADAPTES AU MODELE HUMAIN, PROCEDE DE PREPARATION ET D'UTILISATION

(30) Priority: 27.04.1990 GB 9009548
(43) Date of publication of application: 03.03.1993
(73) Proprietor: CELLTECH THERAPEUTICS LIMITED, Slough, Berkshire SL1 4EN (GB); BOEHRINGER INGELHEIM PHARMACEUTICALS INC., Ridgefield, Connecticut 06877-0368 (US)
(72) Inventor: ADAIR, John, Robert 23 George Road Stokenchurch, Buckinghamshire HP14 3RN (GB); ROBINSON, Martyn, Kim 62 Strawberry Vale, Middlesex TW1 4SE (GB); BRIGHT, Susan, Margaret, Buckinghamshire SL7 2AY (GB); ROTHLEIN, Robert, A., Danbury, CT 06811 (US)
(74) Representative: Laudien, Dieter, Dr.
(86) International application number: US9102946
(87) International publication number: WO9116928

(56) References cited:
- EP-A- 0 289 949
- WO-A-89/01783
- WO-A-91/16927
- US-A- 4 816 567
- BIOTECHNIQUES, vol. 4, no. 3, June 1986, pages 214-221; Oi, V.T. et al.: 'Chimeric antibodies.'
- BIOESSAYS, vol. 8, no. 2, March 1988, CAMBRIDGE, UK, pages 74-78; Verhoeyen, M. et al.: 'Engineering of Antibodies.'
- Proceedings of the National Academy of Science, US, Vol. 86, December 1989; QUEEN et al.: "A Humanized Antibody that Binds to the Interleukin-2 Receptor", pages 10029-10033, see entire document.
- Journal of Immunology, Vol. 143, No. 4,15 August 1989; BARTON et al.: "The Effect of Anti-Intercellular Adhesion Molecule-1 on Phrobol-Ester-Induced Rabbit Lung Inflammation", pages 1278-1282, see Abstract and Discussion.
- European Journal of Immunology, Vol. 20, December 1990; GEISSLER et al.: "A Monoclonal Antibody Directed Against the Human Intercellular Adhesion Molecule (ICAM-1) Modulates the Release of Tumor Necrosis Factor-alpha, Interferon-alpha and Interleukin-1", pages 2591-2596, see entire document.
- European Journal of Immunology, Vol. 20, February 1990, BUCKLE et al., "Human Memory T Cells Express Intercellular Adhesion Molecule-1 which can be Increased by Interleukin-2 and Interferon-alpha", pages 337-341, see entire document.
- Journal of Clinical Investigation, Vol. 82, November 1988; C.W. SMITH et al.: "Recognition of an Endothelial Determinant for CD18-dependent Human Neutrophil Adherence and Transendothelial Migration", pages 1746-1756, see entire document.
- Proceedings of the National Academy of Sciences, US, Vol. 81, issued November 1984; MORRISON et al.: "Chimeric Human Antibody Molecules: Mouse Antigen- Binding Domains with Human Constant Region Domains", pages 6851-6855, see entire document.
- Nature, Vol. 321, 29 May 1986; JONES et al.: "Replacing the Complementarity-determining regions in a Human Antibody with those from a Mouse", pages 522-525, see entire document.
- Trends in Biotechnology, 10(4), 1992, pages 112-113
- Proc. Natl. Acd. Sci. USA, 86, 1989, pages 10029-10033
- American Biotechnology Laboratory, 13(9), 1995, pages 26-28

## Description

### Field of the Invention:

The present invention relates to a humanized chimeric antibody molecule, having specificity for an antigenic determinant of Intercellular Adhesion Molecule 1 (ICAM-1) and to its therapeutic use.

In the present application, the term "chimeric antibody molecule" is used to describe an antibody molecule having heavy and/or light chains comprising at least the variable regions of heavy and/or light chains derived for one immunoglobulin molecule linked to at least part of a second protein. The second protein may comprise additional antibody constant regions domains derived from a different immunoglobulin molecule or a non-immunoglobulin protein. The term "humanized chimeric antibody molecule" is used to describe a molecule having heavy and light chain variable region domains derived from an immunoglobulin from a non-human species, the remaining immunoglobulin constant region domains of the molecule being derived from a human immunoglobulin. The abbreviation "MAb" is used to indicate a monoclonal antibody.

The present invention also relates to the use of humanized chimeric antibodies capable of binding to ICAM-1 to inhibit intercellular adhesion of cells of granulocyte or macrophage lineage. Such molecules can be used for the preparation of an agent for use in a method for the treatment of specific and non-specific inflammation.

The present invention also relates to a humanized chimeric antibody capable of binding ICAM-1 in the treatment of viral, and particularly rhinoviral disease.

The invention also relates to an agent for use in therapeutic and prophylactic methods for suppressing the infection of leukocytes with HIV, and particularly with HIV-1, in an individual who is exposed to HIV or effected by HIV, and is thus in need of such suppression through the administration of a humanized chimeric antibody capable of binding ICAM-1. It therefore provides a an agent for use in therapy for diseases, such as AIDS (Acquired Immunodeficiency Syndrome) which are caused by the HIV virus.

The invention also relates to an agent for use in a therapeutic method for suppressing the migration of HIV-1 infected cells from the circulatory system using a humanized chimeric antibody capable of binding ICAM-1. It therefore provides an agent for use in a therapy for diseases, such as AIDS (Acquired Immunodeficiency Syndrome) which are caused by the HIV-1 virus.

The present invention relates to the use of a humanized chimeric antibody capable of binding ICAM-1 for the preparation of an agent for use in in the treatment of asthma.

### Background of the Invention

### A. Humanized antibodies

Natural immunoglobulins have been known for many years, as have the various fragments thereof, such as the Fab, (Fab')₂ and Fc fragments, which can be derived by enzymatic cleavage. Natural immunoglobulins comprise a generally Y-shaped molecule having an antigen-binding site towards the free end of each upper arm. The remainder of the structure, and particularly the stem of the Y, mediates the effector functions associated with immunoglobulins.

Natural immunoglobulins have been used in assay, diagnosis and, to a more limited extent, therapy. However, such uses, especially in therapy, have been hindered by the polyclonal nature of natural immunoglobulins. A significant step towards the realization of the potential of immunoglobulins as therapeutic agents was the discovery of techniques for the preparation of monoclonal antibodies of defined specificity (Kohler *et al., Nature 265*:295-497 (1975)). However, most MAbs are produced by fusions of rodent spleen cells with rodent myeloma cells. They are therefore essentially rodent proteins. There are very few reports of the production of human MAbs.

Since most available MAbs are of rodent origin, they are naturally antigenic in humans and thus can give rise to an undesirable immune response termed the HAMA (Human Anti-Mouse Antibody) response. Therefore, the use of rodent MAbs as therapeutic agents in humans is inherently limited by the fact that the human subject will mount a immunological response to the MAb and will either remove it entirely or at least reduce its effectiveness. In practice MAbs of rodent origin may not be used in a patient for more than one or a few treatments as a HAMA response soon develops rendering the MAb ineffective as well as giving rise to undesirable reactions.

Proposals have therefore been made for making non-human MAbs less antigenic in humans. Such techniques can be generically termed "humanization" techniques. These techniques generally involve the use of recombinant DNA technology to manipulate DNA sequences encoding the polypeptide chains of the antibody molecule.

In particular one procedure which has bee proposed for the preparation of humanized antibodies is the so-called chimerization procedures.

Such chimerization procedures involve production of chimeric antibodies in which an antigen binding site comprising the complete variable domains of one antibody is linked to constant domains derived from another antibody. Some early methods for carrying out such a chimerization procedure are described in EP-A-0120694 (Celltech Limited), EP-A-0125023 (Genentech Inc. and City of Hope), EP-A-0171496 (Res. Dev. Corp. Japan), EP-A-0173494 (Stanford University), EP-A-0194276 (Celltech Limited). The latter Celltech application also shows the production of an antibody molecule comprising the variable domains of a mouse MAb, the CH1 and CL domains of a human immunoglobulin, and a non-immunoglobulin derived protein in place of the Fc portion of the human immunoglobulin.

### B. Leukocyte Attachment and Functions

Leukocytes and granulocytes must be able to adhere to cellular substrates in order for an inflammatory response to occur and to properly defend the host against foreign invaders such as viruses, bacteria, and allergens. This fact has become evident from two converging lines of research.

The first line of research involves studies of leukocyte membrane proteins (Wallis, W.J., *et al., J. Immunol. 135*:2323-2330 (1985); Mentzer, S.J., *et al., J. Cell. Physiol. 126*:285-290 (1986); Haskard, D.O., *et al., J. Immunol. 137*:2901-2906 (1986); Harlan, J.M., *et al., Blood 66*:167-178 (1985)). Of particular importance to the process of cellular adhesion is a family of leukocyte membrane proteins known as the "CD18" family or complex. This family consists of three heterodimers (known as "Mac-1," "LFA-1," and "P150,90"), all of which share a common subunit (known as the β subunit) ad a unique subunit (known as the α subunit) (Springer, T.A., *et al., Immunol. Rev. 68*:111-135 (1982); Springer, T., *et al., Fed. Proc. 44*:2660-2663 (1985); Keizer, G., *et al*., *Eur. J. Immunol. 15*:1142-1147 (1985); Sanchez-Madrid, F., *et al., J. Exper. Med. 158*:1785-1803 (1983)).

Monoclonal antibodies against the CD18 family of leukocyte membrane proteins, by acting as antagonists of these proteins, inhibit a multitude of leukocyte adhesion dependent events in vitro. This includes the ability of granulocytes to aggregate in response to appropriate stimuli, the ability of granulocytes to attach to protein coated plastic, the ability of granulocytes to migrate in 2-dimensional agarose assays, and the ability of granulocytes to attach to endothelial cells.

The second line of research results from studies involving individuals, who, due to an inherited flaw in the gene encoding for the common subunit of the CD18 family of leukocyte adhesion molecules, are unable to express any of these adhesion molecules on the surfaces of their cells. Such individuals are said to suffer from "leukocyte adherence deficiency disease" ("LAD") (Anderson, D.C., *et al., Fed. Proc. 44*:2671-2677 (1985); Anderson, D.C., *et al., J. Infect. Dis. 152*:668-689 (1985)). Characteristic features of LAD patients include necrotic soft tissue lesions, impaired pus formation and wound healing, as well as abnormalities of adhesion-dependent leukocyte functions in vitro, and susceptibility to chronic and recurring bacterial infections. Granulocytes from these LAD patients behave in the same defective manner in vitro as do their normal counted in the presence of anti-CD18 monoclonal antibody. That is, they are unable to perform adhesion related functions such as aggregation or attachment to endothelial cells. More importantly, however, is the observation that these patients are unable to mount a normal inflammatory response because of the inability of their granulocytes to attach to cellular substrates. Most remarkable is the observation that granulocytes from these LAD patients are unable to get to sites of inflammation such as skin infections due to their inability to attach to the endothelial cells in the blood vessels near the inflammation lesions. Such attachment is a necessary step for extravasation.

Thus, in summary, the ability of lymphocytes and granulocytes to maintain the health and viability of an animal requires that they be capable of adhering to other cells (such as endothelial cells). Granulocyte-endothelial cell adherence has been found to require cell-cell contacts which involve specific receptor molecules present on the granulocyte cell surface. These receptors enable the leukocyte to adhere to other leukocytes or to endothelial, and other non-vascular cells.

The cell surface receptor molecules of leukocytes have been found to be highly related to one another. Humans whose leukocytes lack these cell surface receptor molecules exhibit chronic and recurring infections, as well as other clinical symptoms. Inflammation reactions are mitigated when leukocytes are unable to adhere in a normal fashion due to the lack of functional adhesion molecules of the CD18 complex. Because leukocyte adhesion is involved in the process through which tissue inflammation arises, an understanding of the process of leukocyte adhesion is of significant value in defining a treatment for specific and non-specific inflammation.

Additionally, since lymphocyte adhesion is involved in the process through which foreign body or tissue is identified and rejected, an understanding of this process is of significant value in the fields of organ transplantation, tissue grafting, allergy and oncology.

### C. The Intercellular Adhesion Molecule ICAM-1 and Cellular Adhesion

The intercellular adhesion molecule ICAM-1 was first identified and partially characterized according to the procedure of Rothlein, R. *et al.* (*J. Immunol. 137*:1270-1274 (1986)). ICAM-1, its preparation, purification, and characteristics are disclosed in WO 90/03400.

ICAM-1 was initially realized as being involved in the process of cellular adhesion between endothetial cells and leukocytes. Cellular adhesion is the process through which leukocytes attach to cellular substrates, such as endothetial cells, in order to migrate from circulation to sites of ongoing inflammation, and properly defend the host against foreign invaders such as bacteria or viruses. An excellent review of the defense system is provided by Eisen, H.W., (*In: Microbiology*, 3rd Ed., Harper & Row, Philadelphia, PA (1980), pp. 290-295 and 381-418).

One of the molecules on the surface of endothelial cells which participates in the adhesion process is ICAM-1. This molecule has been shown to mediate adhesion by binding to molecules of the CD-18, CD-11/18 family of glycoproteins which are present on the cell surfaces of leukocytes (Sanchez-Madrid, F. *et al., J. Exper. Med. 158*:1785-1803 (1983); Keizer, G.D. *et al., Eur. J. Immunol. 15*:1142-1147 (1985)).

Intercellular Adhesion Molecule (ICAM-1) is an inducible cell surface glycoprotein expressed on various cell types including vascular endothelial cells, and is expressed preferentially at sites of inflammation. Since ICAM-1 is the natural binding ligand of LFA-1, ICAM-1-LFA-1 interactions play a central role in cellular adhesion, recruitment of lymphocytes to sites of inflammation and the triggering of lymphocyte functions which contribute to both specific and non-specific inflammation.

### D. The Cellular Receptor for Human Rhinovirus

Abraham *et al. (J. Virol. 51*:340-345 (1984)) discovered that the majority of randomly selected human rhinovirus ("HRV") serotypes were able to bind to the same cellular receptor. A monoclonal antibody was subsequently develop by Colonno *et al.* (Colonno *et al., J. Cell. Biochem. Suppl. 10 (part D)*:266 (1986); Colonno *et al., J. Virol. 57*:7-12 (1986); Colonno *et al.*, European Patent Application Publication No. 169,146) which was capable of blocking attachment of HRV of the major serotype to the surfaces of endothelial cells. The endothelial cell receptor protein recognized by this antibody was isolated and found to be a 90 kd protein (Tomassini *et al., J. Virol. 58*:290-295 (1986) and later shown to be the ICAM-1 molecule (Staunton *et al., Cell 56*:849-854 (1989)).

Treatment of rhinoviral infection, especially infection by the major type human rhinovirus has been proposed using a murine monoclonal antibody directed against the viral receptor, ICAM-1 (EP 391088).

### E. Infection with HIV

HIV infection is the cause of AIDS. Two major variants of HIV have been described: HIV-1 and HIV-2. HIV-1 is prevalent in North America and Europe, in contrast to HIV-2 which is prevalent only in Africa. The viruses have similar structures and encode proteins having similar function. The nucleotide and protein sequences of the genes and gene products of the two variants have been found to have about 40% homology with one another.

HIV infection is believed to occur via the binding of a viral protein (termed "gp120") to a receptor molecule (termed "CD4") present on the surface of T4 ("T helper") lymphocytes (Schnittman, S. M. *et al., J. Immunol. 141*:4181-4186 (1988). The virus then enters the cell and proceeds to replicate, in a process which ultimately results in the death of the T cell. The destruction of an individual's T4 population is a direct result of HIV infection. HIV can be recovered from peripheral blood mononuclear cells and human plasma (*J. Clin. Microbiol. 26*:2371-2376 (1988); *N. Engl. J. Med. 321*:1621-1625 (1989)). Results suggest more viremia than had been previously estimated and a T-cell infection frequency as high as 1%.

The destruction of the T cells results in an impairment in the ability of the infected patient to combat opportunistic infections. Although individuals afflicted with AIDS often develop cancers, the relationship between these cancers and HIV infection is, in most cases, uncertain.

Although the mere replication of the HIV virus is lethal to infected cells, such replication is typically detected in only a small fraction of the T4 cells of an infected individual. Several lines of research have elucidated other mechanisms through which the HIV virus mediates the destruction of the T4 population.

Apart from through HIV replication, HIV infected cells can be destroyed through the action of cytotoxic, killer cells. Killer cells are normally present in humans, and serve to monitor the host and destroy any foreign cells (such as in mismatched blood transfusions or organ transplants, etc.) which may be encountered. Upon infection with HIV, T4 cells display the gp120 molecule on their cell surfaces. Killer cells recognize such T4 cells as foreign (rather than native cells), and accordingly, mediate their destruction.

HIV infection can also lead to the destruction of non-infected healthy cells. Infected cells can secrete the gp120 protein into the blood system. The flee gp120 molecules can then bind to the CD4 receptors of healthy, uninfected cells. Such binding causes the cells to take on the appearance of HIV infected cells. Cytotoxic, killer cells recognize the gp120 bound to the uninfected T4 cells, conclude that the cell is foreign, and mediate the destruction of the T4 cells.

An additional mechanism, and one of special interest to the present invention, with which HIV can cause T4 death is through the formation of "syncytia." A "syncytium" is a multinucleated giant cell, formed from the fusion of as many as several hundred T4 cells. Infection with HIV causes the infected cell to become able to fuse with other T4 cells. Such fusion partners may themselves be HIV infected, or they may be uninfected healthy cells. The syncytium cannot function and soon dies. Its death accomplishes the destruction of both HIV infected and HIV uninfected T4 cells. This process is of special interest to the present invention since it entails the direct cell-cell contact of T4 cells. The ability of HIV-infected cells to form syncytia indicates that such cells acquire a means for fusing with healthy cells. Thus, cell-cell contacts may be of fundamental importance in the process through which HIV infection is transmitted from one cell to another within an individual.

HIV infection, and especially HIV-1 infection, appears to influence cell surface expression of the leukocyte integrins and cellular adherence reactions mediated by these heterodimers (Petit, A.J., *et al., J. Clin. Invest. 79*:188 (1987); Hildreth, J.E.K., *et al., Science 244*:1075 (1989); Valentin, A., *et al., J. Immunology 144*:934-937 (1990); Rossen, R.D., *et al., Trans. Assoc. American Physicians 102*:117-130 (1989)). Following infection with HIV-1, homotypic aggregation of U937 cells is increased, as is cell surface expression of CD18, CD11b (Petit, A.J., *et al., J. Clin. Invest. 79*:188 (1987)). HIV-1 infected U937 cells adhere to IL-1 stimulated endothelium in greater frequency than uninfected U937 cells; this behavior can be suppressed by treating the infected cells with anti-CD18 or anti-CD11a monoclonal antibodies or by treating endothelial substrates with anti-ICAM-1 (Rossen, R.D., *et al., Trans.* *Assoc. American Physicians 102*:117-130 (1989)). Monoclonal antibodies to CD18 or CD11a have also been found to be able to inhibit formation of syncytia involving phytohemagglutinin (PHA)-stimulated lymphoblastoid cells and constitutively infected, CD4-negative T cells (Hildreth, J.E.K., *et al., Science 244*:1075 (1989)). Treatment of only the virus infected cells with anti-CD18, or anti-CD11a monoclonal antibodies was found to have little effect on syncytium formation, suggesting that these antibodies principally protect uninfected target cells from Infection (Hildreth, J.E.K., *et al., Science 244*:1075 (1989); Valentin, A., *et al., J. Immunology 144*:934-937 (1990)). Valentin *et al.* (Valentin, A., *et al., J. Immunology 144*:934-937 (1990)) have recently confirmed these observations by demonstrating that monoclonal antibodies specific for CD18 inhibit syncytia formed when continuous T cell lines are co-cultured with HIV-1 infected U937 cells.

Although the mechanism through which monoclonal antibodies specific for CD18 or CD11a protect susceptible cells from fusing with HIV infected cells remains unknown, and is not necessary to an appreciation of the present invention, studies with radiolabeled gp120 suggest that heterodimers containing CD18 do not provide a binding site for the virus (Valentin, A., *et al., J. Immunology 144*:934-937 (1990)). Thus, HIV infection involves cell-cell interactions, and/or viral-cell interactions which mimic such cell-cell interactions. The cell-cell interactions may result in the transport of cell-free virus or the transport of virus across endothelial barriers within the cytoplasm of infected mononuclear cells. Viral-cell interactions which mimic the cell-cell interactions may facilitate or enable free virus to attach to and/or infect healthy cells.

The present invention thus derives, in part, from the observation that HIV infection, and particularly HIV-1, infection results in increased expression of the CD11a/CD18 heterodimer, and its binding ligand, ICAM-1. This increased expression is significant in that it enhances the ability of HIV-infected T cells to adhere or aggregate with one another (i.e. to undergo "homotypic aggregation"). Since such homotypic aggregation is not observed to occur among quiescent normal leukocytes, this discovery indicates that the expression of the CD11/CD18 receptors and/or ICAM-1 is required for such aggregation. Such adhesion permits HIV-1 to be transmitted from an infected cell to a healthy cell of an individual, and also permits or facilitates infection of healthy cells with free virus.

Since ICAM-1 plays a central role in cell-cell interactions murine monoclonal antibodies that bind to ICAM-1 have been proposed as a method of preventing HIV infection (WO 90/13281).

### F. Migration of HIV Infected Cells

The migration and dissemination of leukocytes is important in protecting an individual from the consequences of infection. These processes, however, are also responsible for the migration and dissemination of viral-infected leukocytes. Of particular concern is the migration and dissemination of leukocytes infected with HIV. The migration of such cells results in the formation of extravascular foci, and may cause tumors and other abnormalities.

Histologic examination of affected organs reveals focal extravascular mononuclear cell infiltrates. Attempts to identify virus-infected cells in such infiltrates in the central nervous system have revealed the presence of HIV-1 infected cells. These studies have shown that HIV-1 resides primarily in monocytes and macrophages, and other cells of this lineage (R.T. Johnson, *et al. FASEB J. 2*:2970 (1988); M.H. Stoler *et al., J. Amer. Med. Assn. 256*:2360 (1986); S. Gartner *et al. J. Amer. Med. Assn. 256*:2365 (1986); S. Gartner *et al. Science 233*:215 (1986)).

The mechanisms which stimulate formation of extravascular infiltrates of HIV-1-infected monocytoid cells have not previously been well defined. The mechanisms may involve either the transport of cell-free virus or the transport of virus across endothelial barriers within the cytoplasm of infected mononuclear cells.

Since infection with HIV-1 stimulates cell surface expression of molecules which facilitate adherence of leukocytes to vascular endothelial cells and the translocation of leukocytes from the blood to extravascular tissue sites (C.W. Smith *et al., J. Clin. Invest. 82*:1746 (1988)) it has been proposed to use antibodies which inhibit cellular migration to prevent the dissemination of HIV infected cells (WO 90/13316).

### G. Asthma: Clinical Characteristics

Asthma is a heterogeneous family of diseases. It is characterized by a hyper-responsiveness of the tracheobronchi to stimuli (McFadden, E.R. *et al*., In: *Harrison's Principles of Internal Medicine*, 10th Ed., Petersdorf, R.G. *et al*., Eds., McGraw-Hill, NY (1983), pages 1512-1519); Kay, A.B., *Allergy and Inflammation*, Academic Press, NY (1987)). Clinically, asthma is manifested by the extensive narrowing of the tracheobronchi, by thick tenacious secretions, by paroxysms of dyspnea, cough, and wheezing. Although the relative contribution of each of these conditions is unknown, the net result is an increase in airway resistance, hyperinflation of the lungs and thorax, abnormal distribution of ventilation and pulmonary blood flow. The disease is manifested in episodic periods of acute symptoms interspersed between symptom-free periods. The acute episodes result in hypoxia, and can be fatal. Approximately 3% of the general world population suffers from the disease.

Two types of asthma have been described: allergic asthma and idiosyncratic asthma. Allergic asthma is usually associated with a heritable allergic disease, such as rhinitis, urticaria, eczema, etc. The condition is characterized by positive wheal-and-flare reactions to intradermal injections of airborne antigens (such as pollen, environmental or occupational pollutants, etc.), and increased serum levels of IgE. The development of allergic asthma appears to be causally related to the presence of IgE antibodies in many patients. Asthma patients who do not exhibit the above-described characteristics are considered to have idiosyncratic asthma.

Allergic asthma is believed to be dependent upon an IgE response controlled by T and B lymphocytes and activated by the interaction of airborne antigen with mast cell-bound pre-formed IgE molecules. The antigenic encounter must occur at concentrations sufficient to lead to IgE production for a prolonged period of time in order to sensitize an individual. Once sensitized, an asthma patient may exhibit symptoms in response to extremely low levels of antigen.

Asthma symptoms may be exacerbated by the presence and level of the triggering antigen, environmental factors, occupational factors, physical exertion, and emotional stress.

Asthma may be treated with methylxanthines (such as theophylline), beta-adrenergic agonists (such as catecholamines, resorcinols, saligenins, and ephedrine), glucocorticoids (such as hydrocortisone), inhibitors of mast cell degranulation (i.e. chromones such as cromolyn sodium) and anticholinergics (such as atropine).

Asthma is believed to involve an influx of eosinophils ("eosinophilia") into the tissues of the lung (Frigas, E. *et al., J. Allergy Clin. Immunol. 77*:527-537 (1986)).

Insight into the immunological basis of asthma has been gained from bronchoalveolar lavage studies (Godard, P. *et al., J. Allergy Clin. Immunol. 70*:88 (1982)), and studies of respiratory smooth muscle denuded of epithelium (Flavahan, N.A. *et al., J. Appl. Physiol. 58*:834 (1985); Barnes, P. J. *et al., Br. J. Pharmacol. 86*:685 (1985)). Although these studies have not led to the elucidation of the mechanism underlying the immunology of asthma, they have led to the development of a generally accepted hypothesis concerning the immunological etiology of the disease (see, Frigas, E. *et al., J. Allergy Clin. Immunol. 77*:527-537 (1986)).

The hallmarks of the pathology of asthma are a massive infiltration of the lung parenchyma by eosinophils and the destruction of mucociliary capacity. The "eosinophil hypothesis" suggests that eosinophils are attracted to the bronchus in order to neutralize harmful mediators released by the mast cells of the lung. According to the hypothesis eosinophils are attracted to the bronchi where they degranulate to release cytotoxic molecules. Upon degranulation, eosinophils release enzymes such as histaminase, arylsulfatase and phospholipase D which enzymatically neutralize the harmful mediators of the mast cell. These molecules also promote the destruction of the mucociliary apparatus, and thus prevent the clearing of the bronchial secretions, and contribute to the lung damage characteristic of asthma.

Since asthma involves the migration of cells, it has been proposed to use antibodies which inhibit this migration to mitigate the effects of allergens in a subject (WO 90/10453).

### H. Conclusion

It has been previously proposed; to treat leucocyte-mediated inflammation by administering *inter alia* an anti-ICAM-1 antibody to patients suffering from such inflammation (see EP-0289949 and EP-0314863), to treat viral infection by administering *inter alia* an anti-ICAM-1 antibody to patients suffering from such infection (EP 391088), to prevent the infection of a subject with HIV by administering *inter alia* an anti-ICAM-1 antibody (WO 90/13281), to prevent the dissemination of HIV infected cells by administering *inter alia* an anti-ICAM-1 antibody (WO 90/13316), and to administer *inter alia* an anti-ICAM-1 antibody to mitigate the effects of allergens (WO 90/10453).

EP 289949 describes the preparation of a murine monoclonal (R6-5-D6) having specificity for ICAM-1 which is the preferred antibody for the above referenced therapies. Samples of R6-5-D6 have been deposited with the American Type Culture Collection as deposit ATCC HB9580 on 30th October 1987. R6-5-D6 has been deposited with the ATCC under the provisions of Rule 28(4) of the EPC.

Currently available anti-ICAM-1 MAbs, which are the basis of the above described methods of treatment, are murine MAbs and as a result are likely to cause a significant HAMA response if administered in repeat doses to human patients. It would be highly desirable to diminish or abolish this undesirable HAMA response by suitable humanization or other appropriate recombinant DNA manipulation of these potentially highly useful antibodies and thus extend and enlarge their use. It would also be desirable to apply the techniques of recombinant DNA technology to these antibodies to prepare anti-ICAM-1 humanized chimeric antibodies in general.

We have now prepared anti-ICAM-1 chimeric humanized antibody molecules derived from murine MAbs.

### SUMMARY OF THE INVENTION

The present invention provides a humanized chimeric antibody molecule comprising heavy and/or light chain variable regions of an anti-ICAM-1 antibody.

The invention further pertains to the humanized chimeric antibody of the present invention which are detectably labeled.

The present invention further provides DNA coding for a heavy or light chain variable region of a humanized chimeric anti-ICAM-1 antibody.

The invention additionally includes a recombinant DNA molecule capable of expressing the humanized chimeric antibodies of the present invention.

The invention further includes a host cell capable of producing the humanized chimeric antibodies of the present invention when transformed by the recombinant DNA molecules disclosed herein.

The invention additionally includes use of the humanized chimeric antibodies of the present invention as diagnostic or therapeutic agent.

The invention further provides an agent for use in a method for treating inflammation resulting from a response of the specific defense system in a mammalian subject which comprises providing to a subject in need of such treatment an amount of an anti-inflammatory agent sufficient to suppress the inflammation, wherein the anti-inflammatory agent is a humanized chimeric antibody capable of binding to ICAM-1.

The invention further provides an agent for use in a method for treating non-specific inflammation in humans, and other mammals.

In detail, the method for treating inflammation resulting from a response of the specific and non-specific defense system in a mammalian subject comprises providing to a subject in need of such treatment an anti-inflammatory agent, capable of binding to an ICAM-1, in an amount sufficient to suppress the inflammation; wherein the anti-inflammatory agent is a humanized chimeric antibody capable of binding to ICAM-1.

The above-described method for treating inflammation wherein the inflammation is associated with a condition is selected from the group consisting of: adult respiratory distress syndrome; multiple organ injury syndrome secondary to septicemia; multiple organ injury syndrome secondary to trauma; reperfusion injury of myocardial or other tissues; acute glomerulonephritis; reactive arthritis; dermatosis with acute inflammatory components; acute purulent meningitis or other central nervous system inflammatory disorders such as stroke; thermal injury; hemodialysis; leukapheresis; ulcerative colitis; Crohn's disease; necrotizing enterocolitis; graulocyte transfusion associated syndrome; and cytokine-induced toxicity.

The invention further provides an agent for use in a method of suppressing the metastasis of a hematopoietic tumor cell, the cell requiring a functional member of the LFA-1 family for migration, wherein said method comprises providing to a patient in need of such treatment an amount of an anti-inflammatory agent sufficient to suppress the metastasis; wherein the anti-inflammatory agent is a humanized chimeric antibody capable of binding to ICAM-1.

The invention further provides an agent for use in a method of suppressing the growth of an ICAM-1-expressing tumor cell which comprises providing to a patient in need of such treatment an amount of a toxin sufficient to suppress the growth, the toxin being derivatized to one of the chimeric antibodies of the present invention.

The invention further provides an agent for use in a method of diagnosing the presence ad location of an inflammation resulting from a response of the specific defense system in a mammalian subject suspected of having the inflammation which comprises:
(a) administering to the subject a composition containing a detectably labeled chimeric antibody capable of identifying a cell which expresses ICAM-1, and
(b) detecting the binding ligand.

The invention additionally provides an agent for use in a method of diagnosing the presence and location of an inflammation resulting from a response of the specific defense system in a mammalian subject suspected of having the inflammation which comprises:
(a) incubating a sample of tissue of the subject with a composition containing a detectably labeled chimeric antibody capable of identifying a cell which expresses ICAM-1, and
(b) detecting the binding ligand.

The invention further provides an agent for use in a method of diagnosing the presence and location of an ICAM-1-expressing tumor cell in a mammalian subject suspected of having such a cell, which comprises:
(a) administering to the subject a composition containing a detectably labeled chimeric antibody capable of binding to ICAM-1, and
(b) detecting the binding ligand.

The invention further provides an agent for use in a method of diagnosing the presence and location of an ICAM-1-expressing tumor cell in a mammalian subject suspected of having such a cell, which comprises:
(a) incubating a sample of tissue of the subject with a composition containing a detectably labeled chimeric antibody capable of binding ICAM-1, and
(b) detecting the binding ligand.

The invention additionally includes a pharmaceutical composition comprising:
(a) an anti-inflammatory agent consisting of a humanized chimeric antibody capable of binding to ICAM-1, and
(b) at least one immunosuppressive agent selected from the group consisting of: dexamethasone, azathioprine and cyclosporin A.

The present invention also relates to the use of humanized chimeric antibodies capable of binding ICAM-1 for the preparation of an agent for use in in anti-viral therapy.

In detail, the method for treating viral infection, wherein said virus binds to the ICAM-1 receptor, in an individual in need of such treatment, comprises providing to the individual an amount of a humanized chimeric antibody capable of binding ICAM-1 sufficient to suppress viral infection.

The invention further provides an agent for use in a method for suppressing the infection of leukocytes with HIV, which comprises administering to a patient exposed to or effected by HIV, an effective amount of an HIV-1 infection suppression agent, the agent being a humanized chimeric antibody capable of binding to ICAM-1. In the above method, the HIV can be HIV-1.

The invention further provides an agent for use in a method for suppressing the extravascular migration of a virally infected leukocyte in a patient having such a leukocyte, which comprises administering to the patient an effective amount of a humanized chimeric antibody capable of impairing the ability of said leukocyte to bind to ICAM-1.

In the above-described method, the virally infected leukocytes can be infected with HIV.

In the above-described method, the agent can be a humanized chimeric antibody capable of binding to ICAM-1.

The invention further provides an agent for use in a method for treating asthma in a patient which comprises providing to the patient an effective therapeutic amount of a humanized chimeric antibody capable of binding to ICAM-1

In the above described methods, the chimeric antibody capable of binding to ICAM-1 can be derived from the murine monoclonal antibody R6-5-6D.

### Brief Description of the Figures

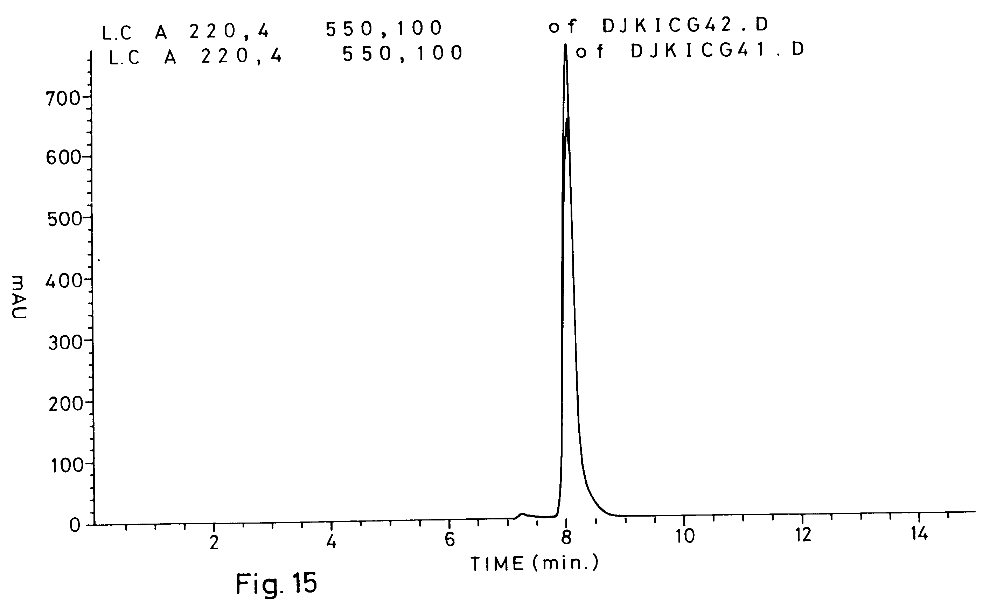

- Figure 1: shows the cDNA sequence for the 5' untranslated region, signal sequence, variable region and part constant region for the R6-5-D6 murine MAb light chain;
- Figure 2: shows similar cDNA and amino acid sequence for the R6-5-D6 murine MAb heavy chain;
- Figure 3: shows a plasmid diagram of plasmid expression vector pEE6 hCMV;
- Figure 4: shows plasmid diagrams indicating the strategy for construction of light chain expression plasmid pAL5;
- Figure 5: shows plasmid diagrams indicating the strategy for construction of heavy chain expression plasmid pAL6;
- Figure 6: shows a graph giving results of a competition assay comprising binding of recombinant and murine R6-5-D6 and a control MAb UPC10;
- Figure 7: shows plasmid diagrams indicating the strategy for the construction of chimeric light chain expression vector pAL7;
- Figure 8: shows plasmid diagrams indicating the strategy for the construction of chimeric heavy chain (IgG2 isotype) expression vector pAL8;
- Figure 9: shows outline restriction maps for the chimeric heavy chain expression vectors pAL8 and pAL9;
- Figure 10: shows plasmid diagrams indicating the procedures involved in the construction of the GS amplification chimeric light chain expression vector pAL10;
- Figure 11: shows similar plasmid diagrams for the construction of the GS chimeric heavy chain (IgG2 isotype) expression vector pAL12;
- Figure 12: shows similar diagrams for the chimeric heavy chain (IgG4 isotype);
- Figure 13: shows SDS-PAGE analysis under non-reducing and reducing conditions. The notation above each lane describes the type of gene used in the transient expression experiment.
mL mouse light cL chimeric light
γ₄ chimeric γ₄ heavy mH mouse heavy
γ₂ chimeric γ₂ heavy B72.3 control cL/cH genes
- Figure 14: shows SDS-PAGE analysis of purified chimeric anti-ICAM-1 antibody or (A) non-reducing and (b) reducing gels.
- On each gel Lane 1: is control chimeric B72.3 antibody (IgG4)
- Lane 2: is Pharmacia low molecular weight markers
- Lanes 3-9: are chimeric anti-ICAM IgG2 4µg → 0.125µg in doubling dilutions
- Lane 10: is Pharmacia low molecular weight markers
- Lanes 11-17: are chimeric anti-ICAM IgG4 4µg → 0.125µg in doubling dilutions
- Figure 15: shows HPLC Gel filtration of chimeric anti-ICAM antibody of IgG2 and IgG4 isotypes.
The profiles are superimposable and elute at a time which corresponds to 150kd tetrameric antibody.

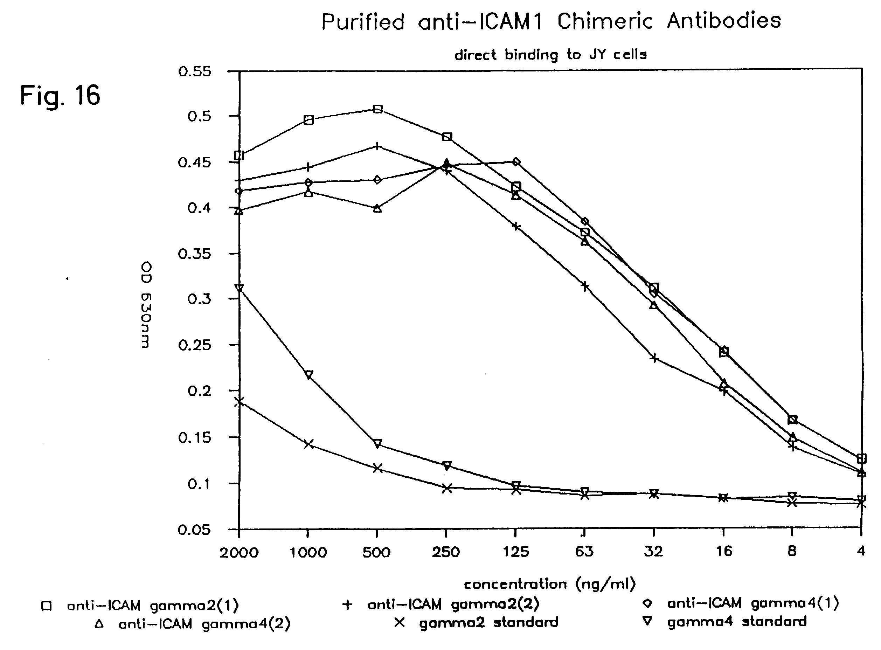

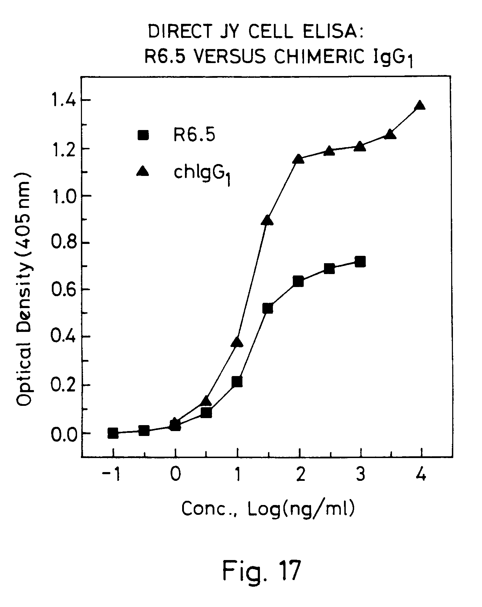

- Figures 16 and 17: shows graphs of binding assays of chimeric antibodies against standards, and

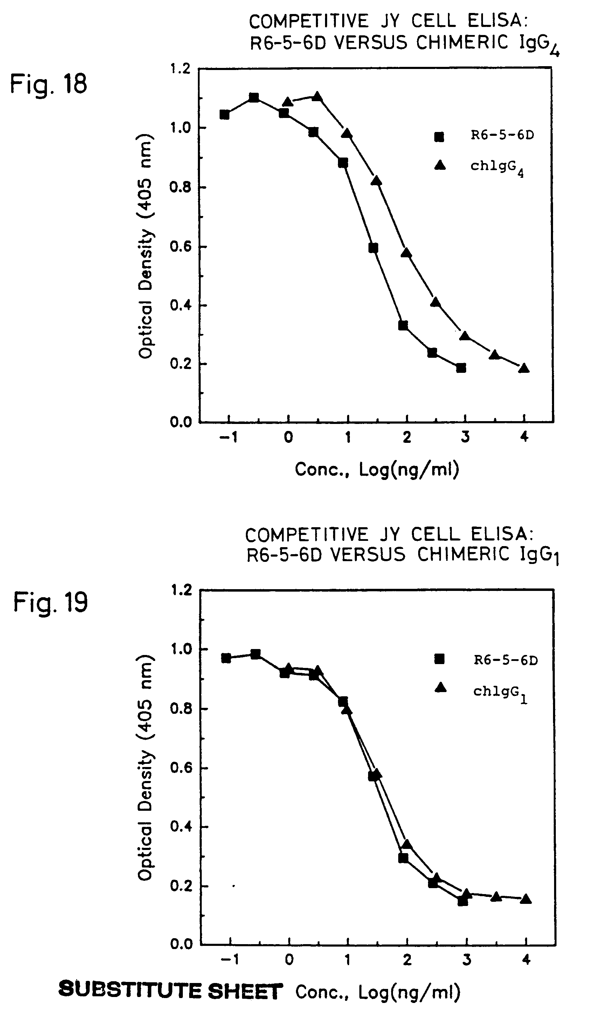

- Figures 18 19 and 20: shows graphs of competition binding assays of chimeric antibodies against standards.

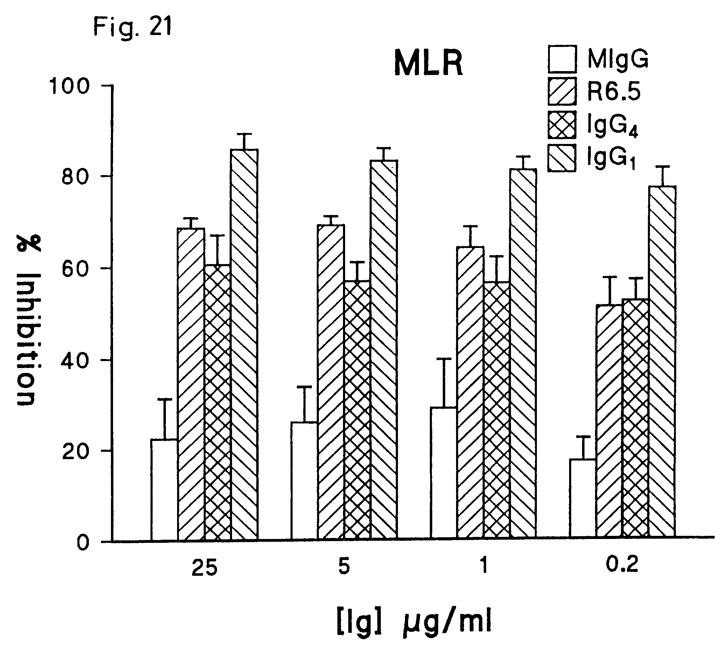

- Figure 21: Inhibition of MLR with antibodies.

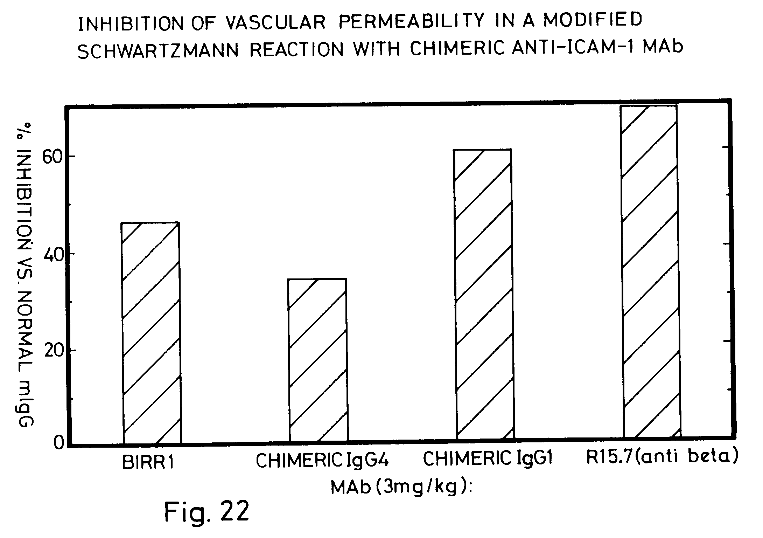

- Figure 22: Inhibition of Vascular permeability in a modified Schwartzmann Assay with antibodies.

### Brief Description of the Preferred Embodiments

### A. Humanized Antibodies

The first embodiment of the present invention provides a humanized chimeric antibodies molecule comprising heavy and/or light chain variable regions of an anti-ICAM-1 antibody according to the claims.

The DNA which codes for such humanized chimeric heavy and/or light chains comprises DNA coding for the non-human liable region domains linked to DNA coding for an IgG1 human constant region domains.

The chimeric antibodies molecule of the present invention may comprise: a complete antibody molecule, having full length heavy and light chains; a fragment thereof, such as the Fab or (Fab')₂ fragment; a light chain or heavy chain monomer or dimer, including fragments thereof or any chimeric antibody molecule with the same specificity as an anti-ICAM-1 antibody.

The chimeric antibodies of the present invention may be a "chemical derivative" of the antibody. As used herein, a molecule is said to be a "chemical derivative" of another molecule when it contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed in *Remington's Pharmaceutical Sciences* (1980). "Toxin-derivatized" molecules constitute a special class of "chemical derivatives." A "toxin-derivatized" molecule is a molecule (such as ICAM-1 or an antibody) which contains a toxin moiety. The binding of such a molecule to a cell brings the toxin moiety into close proximity with the cell and thereby promotes cell death. Any suitable toxin moiety may be employed; however, it is preferable to employ toxins such as, for example, the ricin toxin, the diphtheria toxin, radioisotopic toxins, membrane-channel-forming toxins, etc. Procedures for coupling such moieties to a molecule are well known in the art. Alternatively the chimeric antibody can be attached to a macrocycle, for chelating a heavy metal atom.

Alternatively, the procedures of recombinant DNA technology may be used to produce a "chemical derivative" of the chimeric antibody in which the Fc fragment or CH3 domain of a complete antibody molecule has been replaced by or has attached thereto by peptide linkage a functional non-immunoglobulin protein such as an enzyme or toxin molecule.

In the humanized chimeric antibody molecules, the remainder of the molecule may be derived from any suitable human immunoglobulin. Human constant region domains may be selected having regard to the proposed function of the antibody in particular the effector functions which may be required. For example, the constant region domains may be human IgA, IgE, IgG or IgM domains. In particular, IgG human constant region domains may be used including any of the IgG1, IgG2, IgG3 and IgG4 isotypes. Thus IgG2 or preferably IgG4 isotypes may be used when the humanized chimeric antibody is intended for therapeutic purposes, requiring a absence of antibody effector functions *e.g.,* to block ICAM-1-LFA-1 interactions. Chimeric IgG anti-ICAM-1 antibodies may have different avidities depending on isotype and this may influence the therapeutic choice. Most preferably IgG1 human constant region domains are used. We have found that a IgG1 chimeric antibody appears to have a higher binding avidity for ICAM-1 than IgG2 or IgG4 chimeric antibodies, possibly due to a greater flexibility of the IgG1 hinge promoting bivalent binding to the antigen.

The remainder of the humanized chimeric antibody molecule need not comprise only protein sequences from the human immunoglobulin. For instance, a gene may be constructed in which a DNA sequence encoding part of a human immunoglobulin chain is fused to a DNA sequence encoding the amino acid sequence of a polypeptide effector or reporter molecule.

Preferably, the chimeric antibody molecule of the present invention will be produced by recombinant DNA technology.

A second embodiment of the present invention provides DNA coding for a heavy or light chain variable region of a humanized chimeric antibody according to the claims.

The DNA in the coding sequences for the light and heavy chains may comprise cDNA or genomic DNA or both. However, it is preferred that the DNA sequence encoding the heavy or light chain comprises at least partially genomic DNA. Most preferably, the heavy or light chain encoding sequence comprises a fusion of cDNA and genomic DNA.

Thus, the present invention also includes cloning and expression vectors and transfected cell lines used in the process of the invention.

The general methods by which the vectors may be constructed, transfection methods and culture methods are well know*n per se* and form no part of the invention. Such methods are shown, for instance, in Maniatis *et al.*, *Molecular Cloning*, Cold Spring Harbor, New York (1982); and Primrose and Old, *Principles of Gene Manipulation*, Blackwell, Oxford (1980).

The anti-ICAM-1 antibodies of the invention include all anti-ICAM-1 specificities. Typically, however, the antibodies have specificity for antigenic epitopes of ICAM-1 which when bound by the antibody block, inhibit or otherwise modify ICAM-1/LFA-1 and or ICAM-1/Mac-1 interactions. Preferably the antibodies have specificity for the same or similar ICAM-1 antigenic epitopes as the R6-5-D6 etc. antibodies. Most especially the antibodies are derived from the R6-5-D6 antibody.

### B. Therapeutics and Diagnosis

The present invention also includes therapeutic and diagnostic compositions containing the chimeric antibodies of the invention and uses of such compositions for the preparation of an agent for use in in therapy and diagnosis.

The therapeutic uses to which the products of the anti-ICAM-1 invention may be put include any of the therapeutic uses to which anti-ICAM-1 antibodies may be put including for example any or all of the therapeutic uses described in EP-0289949, EP-0314863, and corresponding applications.

### 1. Anti-Inflammatory Agents

### A. Specific Inflammation

Monoclonal antibodies to members of the CD 18 or CD-11/18 complex inhibit many adhesion dependent functions of leukocytes including binding to endothelium (Haskard, D., *et al., J. Immunol. 137*:2901-2906 (1986)), homotypic adhesions (Rothlein, R., *et al., J. Exp. Med. 163*:1132-1149 (1986)), antigen and mitogen induced proliferation of lymphocytes (Davignon, D., *et al., Proc. Natl. Acad. Sci., USA 78*:4535-4539 (1981)), antibody formation (Fischer, A., *et al., J. Immunol. 136*:3198-3203 (1986)), and effector functions of all leukocytes such as lytic activity of cytotoxic T cells (Krensky, A.M., *et al., J. Immunol. 132*:2180-2182 (1984)), macrophages (Strassman, G., *et al., J. Immunol. 136*:4328-4333 (1986)), and all cells involved in antibody-dependent cellular cytotoxicity reactions (Kohl, S., *et al., J. Immunol. 133*:2972-2978 (1984)). In all of the above functions, the antibodies inhibit the ability of the leukocyte to adhere to the appropriate cellular substrate which in turn inhibits the final outcome. Although both polyclonal and monoclonal antibodies may be employed to inhibit these function, the present invention provides an improvement through the use of a chimeric anti-ICAM-1 antibody.

As discussed previously, the binding of ICAM-1 molecules to the members of LFA-1 family of molecules is of central importance in cellular adhesion. Through the process of adhesion, lymphocytes are capable of continually monitoring an animal for the presence of foreign antigens. Although these processes are normally desirable, they are also the cause of organ transplant rejection, tissue graft rejection and many autoimmune diseases. Hence, any means capable of attenuating or inhibiting cellular adhesion would be highly desirable in recipients of organ transplants, (e.g., kidney), tissue grafts or autoimmune patients.

A humanized chimeric antibody capable of binding to ICAM-1 is highly suitable as an anti-inflammatory agent in a mammalian subject. Significantly, such an agent differs from general anti-inflammatory agents and non-humanized antibodies in that they are capable of selectively inhibiting adhesion, do not offer other side effects such as nephrotoxicity which are found with conventional agents, and limit the amount of HAMA associated with the use of murine MAbs. A humanized chimeric antibody capable of binding to ICAM-1 can therefore be used to prevent organ (e.g. kidney) or tissue rejection, or modify autoimmune responses without the fear of such side effects, in the mammalian subject.

Importantly, the use of humanized antibodies capable of recognizing ICAM-1 may permit one to perform organ transplants even between individuals having HLA mismatch.

In the forth embodiment of the present invention an agent for use in a method for suppressing specific inflammation is provided wherein said method comprises providing to recipient subjects in need of such a treatment an amount of one of the humanized chimeric antibodies of the present invention sufficient to suppress inflammation. An amount is said to be sufficient to "suppress" inflammation if the dosage, route of administration, etc. of the agent are sufficient to attenuate or prevent inflammation.

The humanized chimeric antibody may be administered either alone or in combination with one or more additional immunosuppressive agents (especially to a recipient of an organ (e.g. kidney) or tissue transplant). The administration of such a composition may be for either a "prophylactic" or "therapeutic" purpose. When provided prophylactically, the immunosuppressive composition is provided in advance of any inflammatory response or symptom (for example, prior to, at, or shortly after) the time of an organ or tissue transplant but in advance of any symptoms of organ rejection). The prophylactic administration of the composition serves to prevent or attenuate any subsequent inflammatory response (such as, for example, rejection of a transplanted organ or tissue, etc.). When provided therapeutically, the immunosuppressive composition is provided at (or shortly after) the onset of a symptom of actual inflammation (such as, for example, organ or tissue rejection). The therapeutic administration of the composition serves to attenuate any actual inflammation (such as, for example, the rejection of a transplanted organ or tissue).

The anti-inflammatory agents of the present invention may, thus, be provided either prior to the onset of inflammation (so as to suppress an anticipated inflammation) or after the initiation of inflammation.

Since ICAM-1 molecules are expressed mostly at sites of inflammation, such as those sites involved in delayed type hypersensitivity reaction, antibodies (especially chimeric antibodies derived from anti-ICAM-1 monoclonal antibodies) capable of binding to ICAM-1 molecules have therapeutic potential in the attenuation or elimination of such regions. This potential therapeutic use may be exploited in either of two manners. First, a composition containing a chimeric antibody capable of binding to ICAM-1 may be administered to a patient experiencing delayed type hypersensitivity reaction. For example, such compositions might be provided to a individual who had been in contact with antigens such as poison ivy, poison oak, etc.

In a sixth embodiment, one of the chimeric antibodies of the present invention is administered to a patient in conjunction with an antigen in order to prevent a subsequent inflammatory reaction. Thus, the additional administration of an antigen with an ICAM-1-binding chimeric antibody may temporarily tolerize an individual to subsequent presentation of that antigen.

Since LAD patients that lack LFA-1 do not mount an inflammatory response, it is believed that antagonism of LFA-1's natural ligand, ICAM-1, will also inhibit an inflammatory response. The ability of antibodies against ICAM-1 to inhibit inflammation provides the basis for their therapeutic use in the treatment of chronic inflammatory diseases and autoimmune diseases such as lupus erythematosus, autoimmune thyroiditis, experimental allergic encephalomyelitis (EAE), multiple sclerosis, some forms of diabetes Reynaud's syndrome, rheumatoid arthritis, etc. Such antibodies may also be employed for the preparation of an agent for use in a therapeutical treatment of psoriasis. In general, a chimeric antibody capable of binding ICAM-1 may be employed in the treatment of those diseases currently treatable through steroid therapy.

### B. Non-specific Inflammation

The present invention derives from the discovery that ICAM-1 on endothelial cells binds to the members of the CD18 family of molecules on granulocytes responsible for mediating granulocyte-endothelial cell adhesion and that antagonists of ICAM-1 are capable of inhibiting such adhesion. Such inhibition provides a means for treating general, non-specific tissue inflammation.

Since cellular adhesion is required in order that leukocytes may migrate to sites of non-specific inflammation and/or carry out various effector functions contributing to the inflammation, agents which inhibit cellular adhesion will attenuate or prevent this inflammation. A "non-specific defense system reaction" is a response mediated by leukocytes incapable of immunological memory. Such cells include granulocytes and macrophages. As used herein, inflammation is said to result from a response of the non-specific defense system, if the inflammation is caused by, mediated by, or associated with a reaction of the non-specific defense system. Examples of inflammation which result, at least in part, from a reaction of the non-specific defense system include inflammation associated with conditions such as: adult respiratory distress syndrome (ARDS) or multiple organ injury syndromes secondary to septicemia or trauma; reperfusion injury of myocardial or other tissues; acute glomerulonephritis; reactive arthritis; dermatoses with acute inflammatory components; acute purulent meningitis or other central nervous system inflammatory disorders, e.g. stroke; thermal injury; hemodialysis; leukapheresis; ulcerative colitis; Crohn's disease; necrotrizing enterocolitis; granulocyte transfusion associated syndromes; and cytokine-induced toxicity.

In a fifth embodiment of the present invention an agent for use in a method of treating non-specific inflammation is provided wherein said method comprises providing to a subject in need of such treatment an effective amount of one of the chimeric antibody of the present invention.

### 2. Diagnostic and Prognostic Applications

Since ICAM-1 is expressed mostly at sites of inflammation, a chimeric antibody capable of binding ICAM-1 may be employed as a means of imaging or visualizing the sites of infection and inflammation in a patient.

In an eighth embodiment of the present invention, a chimeric antibody of the present invention is detectably labeled, through the use of radioisotopes, affinity labels (such as biotin, avidin, etc.) fluorescent labels, paramagnetic atoms, etc and are used as an agent to be provided to a patient to localize the site of infection or inflammation. Procedures for accomplishing such labeling are well known to the art. Clinical application of antibodies in diagnostic imaging are reviewed by Grossman, H.B., *Urol. Clin. North Amer. 13*:465-474 (1986)), Unger, E.C. *et al., Invest. Radiol. 20*:693-700 (1985)), and Khaw, B.A. *et al., Science 209*:295-297 (1980)).

The detection of foci of such detectably labeled antibodies is indicative of a site of inflammation or tumor development. In one embodiment, this examination for inflammation is done by removing samples of tissue, including blood cells, and incubating such samples in the presence of the detectably labeled antibodies. In a preferred embodiment, this technique is done in a non-invasive manner through the use of magnetic imaging, fluorography, etc. Such a diagnostic test may be employed in monitoring organ transplant recipients for early signs of potential tissue rejection. Such assays may also be conducted in efforts to determine an individual's predilection to rheumatoid arthritis or other chronic inflammatory diseases.

### 3. Adjunct to the Introduction of Antigenic Material Administered for Therapeutic or Diagnostic Purposes

Immune responses to therapeutic or diagnostic agents such as, for example, bovine insulin, interferon, tissue-type plasminogen activator or murine monoclonal antibodies substantially impair the therapeutic or diagnostic value of such agents, and can, in fact, causes diseases such as serum sickness. Such a situation can be remedied through the use of the chimeric antibodies of the present invention. In this embodiment, such antibodies would be administered in combination with the therapeutic or diagnostic agent.

In a ninth embodiment, the addition of an effective amount of a chimeric antibody of the present invention with specificity to ICAM-1 is used as an agent to be administered to a subject in order to prevent the recipient from recognizing the agent, and therefore prevent the recipient from initiating an immune response against it. The absence of such an immune response results in the ability of the patient to receive additional administrations of the therapeutic or diagnostic agent.

### 4. Anti-viral usage of chimeric antibodies according to the invention

Another aspect of the present invention relates to the discovery of that ICAM-1 is the cellular receptor of certain viruses, and is thus required in order for the virus to adhere to and infect human cells (Greve, J.M. *et al., Cell 56*:839-847 (1989); Staunton, D.E. *et al., Cell 56*:849-853 (1989)). In particular, rhinoviruses, and especially rhinoviruses of the major serotype have been found to be capable of mediating their infection through their capacity to bind to the ICAM-1 molecules present on cell surfaces.

The tenth embodiment of the present invention is directed toward the use of chimeric antibodies of the present invention capable of binding ICAM-1 as an agent to treat viral infection. Because such antibodies are capable of blocking the ICAM-1 of endothelial cells for viral attachment, their administration to a recipient individual results in the decrease in receptors available for viral binding, and thus decreases the percentage of viruses which attach and infect the cells of an individual.

ICAM-1 has the ability to interact with and bind to viruses, and in particular, rhinoviruses of the major serotype within the genus Picornaviridae, group A coxsackieviruses (Colonno, R.J. *et al., J. virol. 57*:7-12 (1986)) and Mengo viruses (Rossmann, M.G. *et al., Virol. 164*:373-382 (1988)). This interaction is mediated by ICAM-1 amino acid residues which are present in domain 1 of the ICAM-1 molecule. Such interactions are assisted, however, by contributions from amino acids present in domains 2 and 3 of ICAM-1. Thus, among the preferred chimeric antibodies of this embodiment are antibodies capable of binding to domains 1, 2, and 3 of ICAM-1. More preferred are chimeric antibodies capable of binding to domains 1 and 2 of ICAM-1. Most preferred are chimeric antibodies capable of binding domain 1 of ICAM-1.

The administration of the anti-viral agents of the present invention may be for either a "prophylactic" or "therapeutic" purpose. When provided prophylactically, the anti-viral agent is provided in advance of any symptom of viral infection (for example, prior to, at, or shortly after the time of infection, but in advance of any symptoms of such infection). The prophylactic administration of the agent serves to prevent or attenuate any subsequent viral infection, or to reduce the possibility that such infection will be contagious to others.

When provided therapeutically, the anti-viral agent is provided at (or shortly after) the onset of a symptom of actual viral infection (such as, for example, nasal congestion, fever, etc). The therapeutic administration of the agent serves to attenuate any actual viral infection.

The anti-viral agents of the present invention may, thus, be provided either prior to the onset of viral infection (so as to suppress an anticipated infection) or after the initiation of such infection.

### 5. Suppression of HIV Infection and the prevention of the Dissemination of HIV Infected Cells.

The eleventh embodiment of present invention provides an agent for use in a method for suppressing the infection of HIV, which comprises administering to an HIV-infected individual an effective amount of an HIV infection suppression agent. Although the invention is particularly concerned with an agent for use in a method for the suppression of HIV-1 infection, it is to be understood that the method may be applied to any HIV-1 variant (such as, for example, HIV-2) which may infect cells in a way which may be suppressed by the agents of the present invention. Such variants are the equivalents of HIV-1 for the purposes of the present invention.

One aspect of the present invention derives from the recognition that expression of LFA-1 and, in some cases, ICAM-1, stimulated by HIV infection, promotes cell-to-cell adherence reactions that can increase the contact time of infected with uninfected cells, facilitating transfer of virus from infected to uninfected cells. Thus, chimeric antibodies capable of binding ICAM-1 are able to suppress infection by HIV, and, in particular, by HIV-1.

One means through which molecules which bind to ICAM-1 may suppress HIV infection is by impairing the ability of the ICAM-1 expressed by HIV-infected cells to bind to the CD11/CD18 receptors of a healthy T cell. In order to impair the ability of a cell to bind to the CD11a/CD18 receptor, or to the ICAM-1 ligand molecule, it is possible to employ chimeric antibodies of the present invention capable of binding to ICAM-1.

The agents of the present invention are intended to be provided to recipient subjects in an amount sufficient to achieve a suppression of HIV infection. An amount is said to be sufficient to "suppress" HIV infection if the dosage, route of administration, etc. of the agent are sufficient to attenuate or prevent such HIV infection. The agents are to be provided to patients who are exposed to, or effected by HIV infection.

The chimeric antibodies of the present invention may be for either a "prophylactic" or "therapeutic" purpose in the treatment of HIV infection. When provided prophylactically, the antibody is provided in advance of any symptom of viral infection (for example, prior to, at, or shortly after the time of such infection, but in advance of any symptoms of such infection). The prophylactic administration of the antibody serves to prevent or attenuate any subsequent HIV infection. When provided therapeutically, the antibody is provided at (or shortly after) the detection of virally infected cells. The therapeutic administration of the antibody serves to attenuate any additional HIV infection.

The agents of the present invention may, thus, be provided either prior to the onset of viral infection (so as to suppress the anticipated HIV infection) or after the actual detection of such virally infected cells (to suppress further infection).

In particular, the invention provides an improved agent for use in a therapy for AIDS, and an enhanced means for suppressing HIV infection, and particularly HIV-1 infection, which comprises the co-administration of:
(I) ICAM-1, a soluble ICAM-1 derivative, CD11 (either CD11a, CD11b, or CD11c), a soluble CD11 derivative, CD18, a soluble CD18 derivative, or a CD11/CD18 heterodimer, or a soluble derivative of a CD11/CD18 heterodimer and/or
(II) a chimeric antibody of the present invention capable of binding to ICAM-1 with
(III) cell or particle associated CD4 or a soluble derivative of CD4 and/or
(IV) a molecule (preferably an antibody or antibody fragment) capable of binding to CD4.

In the twelfth embodiment of the present invention also an agent for use in a method for suppressing the migration of HIV-infected cells is provided wherein said method comprises administering an effective amount of an anti-migration agent to an HIV-infected individual.

The anti-migration agents of the present invention include any chimeric antibody capable of imparting the ability of an HIV-infected T cell to bind to ICAM-1. Chimeric antibodies which bind to ICAM-1 will suppress migration by impairing the ability of the ICAM-1 expressed by HIV-infected T cells to bind to cells expressing a CD11/CD18 receptor. In order to impair the ability of a cell to bind to the CD11a/CD18 receptor it is possible to employ a chimeric antibody capable of binding to ICAM-1.

The agents of the present invention are intended to be provided to recipient subjects in an amount sufficient to suppress the migration of HIV (or other virally) infected T cells. An amount is said to be sufficient to "suppress" migration of T cells if the dosage, route of administration, etc. of the agent are sufficient to attenuate or prevent such migration.

The administration of a chimeric antibody may be for either a "prophylactic" or "therapeutic" purpose. When provided prophylactically, the chimeric antibody is provided in advance of any symptom of viral infection (for example, prior to, at, or shortly after) the time of such infection, but in advance of any symptoms of such infection). The prophylactic administration of the chimeric antibody serves to prevent or attenuate any subsequent migration of virally infected T cells. When provided therapeutically, the chimeric antibody is provided at (or shortly after) the detection of virally infected T cells. The therapeutic administration of the antibody serves to attenuate any additional migration of such T cells.

The chimeric antibodies of the present invention may, thus, be provided either prior to the onset of viral infection (so as to suppress the anticipated migration of infected T cells) or after the actual detection of such virally infected cells.

### 6. Treatment of Asthma

In the thirteenth embodiment of the present invention a chimeric antibody capable of binding to ICAM-1 is used as an agent for use in the treatment of asthma.

The therapeutic effects of the anti-asthma agents of the present invention may be obtained by providing such agents to a patient by any suitable means (i.e. intravenously, intramuscularly, subcutaneously, enterally, or parenterally). It is preferred to administer the agents of the present invention intranasally as by nasal spray, swab, etc. It is especially preferred to administer such agents by oral inhalation, or via an oral spray or oral aerosol. When administering agents by injection, the administration may be by continuous infusion, or by single or multiple boluses.

The anti-asthma agents of the present invention are intended to be provided to recipient subjects in an amount sufficient to lessen or attenuate the severity, extent or duration of the asthma symptoms.

The chimeric antibodies of the present invention may be administered either alone or in combination with one or more additional anti-asthma agents (such as methylxanthines (such as theophylline), beta-adrenergic agonists (such as catecholamines, resorcinols, saligenins, and ephedrine), glucocorticoids (such as hydrocortisone), chromones (such as cromolyn sodium) and anticholinergics (such as atropine), in order to decrease the amount of such agents needed to treat the asthma symptoms.

The administration of the chimeric antibodies of the present invention may be for other a "prophylactic" or "therapeutic" purpose. When provided prophylactically, the chimeric antibodies are provided in advance of any asthma symptom. The prophylactic administration of the chimeric antibody serves to prevent or attenuate any subsequent asthmatic response. When provided therapeutically, the chimeric antibody is provided at (or shortly after) the onset of a symptom of asthma. The therapeutic administration of the antibody serves to attenuate any actual asthmatic episode. The chimeric antibodies of the present invention may, thus, be provided either prior to the onset of an anticipated asthmatic episode (so as to attenuate the anticipated severity, duration or extent of the episode) or after the initiation of the episode.

### C. Administration of the Compositions of the Present Invention

The therapeutic effects of chimeric antibodies capable of binding ICAM-1 may be obtained by providing to a patient an effective amount of a chimeric antibody which is substantially free of natural contaminants. The chimeric antibodies of the present invention disclosed herein are said to be "substantially free of natural contaminants" if preparations which contain them are substantially free of materials with which these products are normally and naturally found.

The present invention extends to humanized chimeric antibodies which may be produced either by an animal, or by tissue culture, or recombinant DNA means.

In providing a patient with a chimeric antibody of the present invention, the dosage of administered agent will vary depending upon such factors as the patient's age, weight, height, sex, general medical condition, previous medical history, etc. In general, it is desirable to provide the recipient with a dosage of antibody which is in the range of from about 1 pg/kg to 10 mg/kg (body weight of patient), although a lower or higher dosage may be administered.

A chimeric antibody of the present invention capable of binding to ICAM-1 may be administered to patients intravenously, intramuscularly, subcutaneously, enterally, topically inhaled, intranasally, or parenterally. When administering an antibody, the administration may be by continuous administration, or by single or multiple boluses.

A composition is said to be "pharmacologically acceptable" if its administration can be tolerated by a recipient patient. Such an agent is said to be administered in a "therapeutically effective amount" if the amount administered is physiologically significant. An agent is physiologically significant if its presence results in a detectable change in the physiology of a recipient patient.

The chimeric antibodies of the present invention can be formulated according to known methods to prepare pharmaceutically useful compositions, whereby these antibodies are combined in a mixture with a pharmaceutically acceptable carrier vehicle. Suitable vehicles and their formulation, inclusive of other human proteins, e.g., human serum albumin, are described, for example, in Remington's Pharmaceutical Sciences (16th ed., Osol, A., Ed., Mack, Easton PA (1980)). In order to form a pharmaceutically acceptable composition suitable for effective administration, such compositions will contain an effective amount of a chimeric antibody together with a suitable amount of carrier vehicle.

Additional pharmaceutical methods may be employed to control the duration of action. Controlled release preparations may be achieved through the use of polymers to complex or absorb the chimeric antibody. The controlled delivery may be exercised by selecting appropriate macromolecules (for example polyesters, polyamino acids, polyvinyl, pyrrolidone, ethylenevinylacetate, methylcellulose, carboxymethylcellulose, or protamine, sulfate) and the concentration of macromolecules as well as the methods of incorporation in order to control release. Another possible method to control the duration of action by controlled release preparations is to incorporate the chimeric antibody into particles of a polymeric material such as polyesters, polyamino acids, hydrogels, poly(lactic acid) or ethylene vinylacetate copolymers. Alternatively, instead of incorporating the antibody into polymeric particles, it is possible to entrap these materials in microcapsules prepared, for example, by coacervation techniques or by interfacial polymerization, for example, hydroxymethylcellulose or gelatine-microcapsules and poly(methylmethacylate) microcapsules, respectively, or in colloidal drug delivery systems, for example, liposomes, albumin microspheres, microemulsions, nanoparticles, and nanocapsules or in macroemulsions. Such techniques are disclosed in Remington's Pharmaceutical Sciences (1980).

### MATERIAL METHODS

### 1. Incoming Cells

Hybridoma cell line R6-5-D6 producing anti-ICAM-1 antibody was provided by Boehringer Ingelheim Pharmaceuticals Inc. (Lot No. R6-5-D6 - E9-B2 0-29-86) and was grown up in antibiotic free Dulbecco's Modified Eagles Medium (DMEM) supplemented with glutamine and 5% foetal calf serum, and divided to provide both an overgrown supernatant for evaluation and cells for extraction of RNA. The overgrown supernatant was shown to contain murine IgG2a/kappa antibody. Cell culture supernatant was examined and confirmed to contain the antibody R6-5-D6.

### 2. Molecular Biology Procedures

Basic molecular biology procedures were as Maniatis *et al.* (1982) (Maniatis *et al., Molecular Cloning*, Cold Spring Harbor, New York (1982)) with, in some cases, minor modifications. DNA sequencing was performed as described in Sanger *et al.* (1977) (Sanger *et al.*, *Proc. Natl. Acad. Sci.* *USA 74*:5463-5467 (1977)) and the Amersham International Plc sequencing handbook. COS cell expression and metabolic labelling studies were as described in Whittle *et al.* (1987) (Whittle *et al., Prot. Eng. 1, 6*:499-505 (1987)). Chinese Hamster Ovary (CHO) transfections and cell culture were performed as described in Gorman (1988) (Gorman, C. , *DNA Cloning 2*:143-190 *ed.* (1988)) and Bebbington and Hentschel (1988) (Bebbington *et al., DNA Cloning 3*:163-188 *ed.* (1988)).

### 3. Research Assays

### 3.1. Assay for Secreted Antibody Light Chain

Supernatants from CHO cell lines were assayed for secreted light chain, after transfection with light chain expression vectors, as the first step in the development of stable cell lines producing whole chimeric antibody. The procedure was as follows:
96 well microtitre plates were coated with F(ab')₂ goat anti-human kappa light chain. The plates were washed with water and samples added and incubated for one hour at room temperature. The plates were washed and F(ab')₂ goat anti-human F(ab')₂ Horse radish peroxidase (HRPO) conjugate was then added and incubated for a further hour. Enzyme substrate was then added to reveal the reaction.

### 3.2. Assembly Assays

Assembly assays were performed on supernatants from transfected COS cells and from transfected CHO cells to determine the amount of intact IgG present.

### 3.2.1 COS and CHO Cells transfected with mouse genes

The assembly assay for intact mouse IgG in cell supernatants was an ELISA with the following format:
96 well microtitre plates were coated with F(ab')₂ goat anti-mouse IgG Fc. The pates were washed in water and samples added and incubated for 1 hour at room temperature. The plates were washed and F(ab')₂ goat anti-mouse IgG F(ab')₂ (HRPO conjugated ) was then added. Enzyme substrate was then added to reveal the reaction. UPC10, a mouse IgG2a myeloma, was used as a standard.

### 3.2.2 COS and CHO Cells transfected with Chimeric Genes

The assembly assay for intact humanized anti-ICAM-1 in COS cell supernatants was an ELISA with the following format:
96 well microtitre plates were coated with F(ab')₂ goat anti-human IgG Fc. The plates were washed and samples added and incubated for 1 hour at room temperature. The plates were washed and monoclonal mouse anti-human kappa chain was added and incubated for 1 hour at room temperature. The plates were washed and F(ab')₂ goat anti-mouse IgG Fc (HRPO conjugated) was added. Enzyme substrate was then added to reveal the reaction. Chimeric B72.3 (Bodmer *et al*., Published International Patent Application WO 89/01783) (IgG4) and pooled, purified human IgG2 and IgG4 (Chemicon) were used initially as standards. Later, purified chimeric IgG4 anti-ICAM-1 was used is a standard for work with chimeric IgG1 anti-ICAM-1. The use of a monoclonal anti-kappa chain in this assay allows the amount of chimeric antibody to be read from the standards.

### 3.3. Assay for Antigen Binding Activity

### 3.3.1 Direct Binding

Material from COS and CHO cell supernatants and purified chimeric antibodies were assayed for anti-ICAM-1 antigen binding activity onto ICAM-1 positive cells in a direct assay. The procedure was as follows:
JY cells (a human B lymphoblastoid cell line which constitutively expresses ICAM-1 on the cell surface) were maintained in culture. Monolayers of JY cells were fixed onto 96 well. ELISA plates using poly-L-lysine and paraformaldehyde, and the plates were blocked with a solution of bovine serum albumin in PBS. Samples were added to the monolayers and incubated for 1 hour at room temperature. The plates were washed gently using PBS. F(ab')₂ goat anti-human IgG Fc (HRPO conjugated) or F(ab')₂ goat anti-mouse IgG Fc (HRPO conjugated) was then added as appropriate for humanized or mouse samples. Enzyme substrate was then added to reveal the reaction. The negative control for the cell-based assay was chimeric B72.3 (IgG4) or pooled, purified human IgG2 and IgG4 (Chemicon). The positive control was murine R6-5-D6 MAb.

### 3.3.2 Competition Binding

Monolayers of JY cells were prepared as in 3.3.1. Antibody samples were added and incubated overnight at 4°C. Biotinylated anti-ICAM-1 was added to all the wells. The mixture was left at room temperature for 2 hours. The plates were washed and either streptavidin-HRPO or streptavidin-betagalactosidase was added. After further incubation enzyme substrate was added to reveal the reaction.

### 3.3.3 Mixed Lymphocyte Reaction Assays

Peripheral blood was obtained from normal, healthy donors by venipuncture. The blood (7.5 ml) was layered over 7.5 ml of a Ficoll/Hypaque density gradient (Pharmacia, density = 1.078) room temperature and centrifuged at 1000 x g for 20 minutes. The cells were washed, counted on a hemacytometer, and suspended in RPMI-1640 culture medium (Gibco) containing 50 µg/ml gentamycin, 1 nM L-glutamine (Gibco) and 5% heat activated (55 C,30 min) human AB sera (Flow Laboratories) (hereafter referred to as RPMI-culture medium).

Peripheral blood mononuclear cells (responder cells) were cultured in medium at 6.25 X 10⁻⁵ cells/ml in Linbro round-bottomed microtiter plates (#76-013-05). Stimulator cells from a separate donor were irradiated at 0.258 C/kg (1000R) and cultured with the responder cells at the same concentration. Responder cells were added to wells, followed by the monoclonal antibodies and the stimulator cells were added last. The total volume per culture was 0.2 ml. Controls included responder cells alone. The culture plates were incubated at 37°C in a 5% CO₂-humidified air atmosphere for 5 days. The wells were pulsed with 0.5 µCi tritiated thymidine (³HT) (New England Nuclear) for the last 18 hours of culture.

The cells were harvested onto glass fiber filters using an automated multiple sample harvester (Skatron, Norway), rinsing with distilled water. The filters were oven dried and counted in Beckman Ready Safe liquid scintillation cocktail on a LKB Betaplate liquid scintillation counter.

### 3.4 In Vivo Assays

### 3.4.1 Modified Schwartzmann Reaction

The local Schwartzmann reaction in rabbit skin can be produced by an i.d. injection of endotoxin followed by an i.v. challenge injection of zymosan 18-24 hrs. liter. The hemorrhagic necrosis that develops in the previously injected skin sites is characterized by microthrombi, intravascular neutrophil aggregation, platelet and fibrin deposition, vascular permeability increases and massive extravasation of erythrocytes (RBC). We have modified this protocol for use in the Cynomologous monkey. Separate, distinct skin sites were injected i.d. with endotoxin (3 µg/site) or normal saline and 18 hours later these same sites were injected with zymosan (300 µg/site). The resulting inflammatory response was quantitated at 6 hours post-zymosan by measuring the increase in vascular permeability in the endotoxin-injected sites compared to the saline-injected sites using ¹²⁵I-BSA. The inhibitory effect of R6.5 (anti-ICAM-1), the IgG1, IgG2, and IgG4, chimerics of R6.5 and R15.7 (LFA-1 beta) were compared to that of normal mouse IgG. All IgG preparations were administered i.v. at 3 mg/kg prior to the zymosan injections.

### Results

### 4. CDNA Library Construction

### 4.1 mRNA Preparation and cDNA Synthesis

Cells were grown as described in Section 1 and 1.4 x 10⁹ cells harvested and mRNA extracted using the guanidinium/LiCl extraction procedure. cDNA was prepared by priming from Oligo-dT to generate full length cDNA. The cDNA was methylated and EcoRI linkers added for cloning.

### 4.2 Library Construction

The cDNA library was ligated to pSP64 vector DNA which had been EcoRI cut ad the 5' phosphate groups removed by calf intestinal phosphatase (EcoRI/CIP). The ligation was used to transform high transformation efficiency *Escherichia coli* HB101 (*E. coli* HB101) from Bethesda Research Labs (BRL) in the case of the light chain and *E. coli* LM1035 prepared by electroporation (Dower *et al., Nucl. Acids Res. 16*:6127 (1988)) in the case of the heavy chain. cDNA libraries were prepared. 11600 colonies were screened for the light chain ad 25000 colonies were screened for the heavy chain.

### 5. Screening

*E. coli c*olonies positive for either heavy or light chain probes were identified either by oligonucleotide screening using the oligonucleotide: 5' TCCAGATGTTAACTGCTCAC for the light chain, which is complementary to a sequence in the mouse kappa constant region, or by using a 980 bp BamHI-EcoRI restriction fragment of a previously isolated mouse IgG2a constant region clone. 6 light chain and 10 heavy chain clones were identified and taken for second round screening. Positive clones from the second round of screening were grown up and DNA prepared. The sizes of the gene inserts were estimated by gel electrophoresis and DNA inserts of a size capable of containing a full length cDNA were sequenced.

### 6. DNA Sequencing

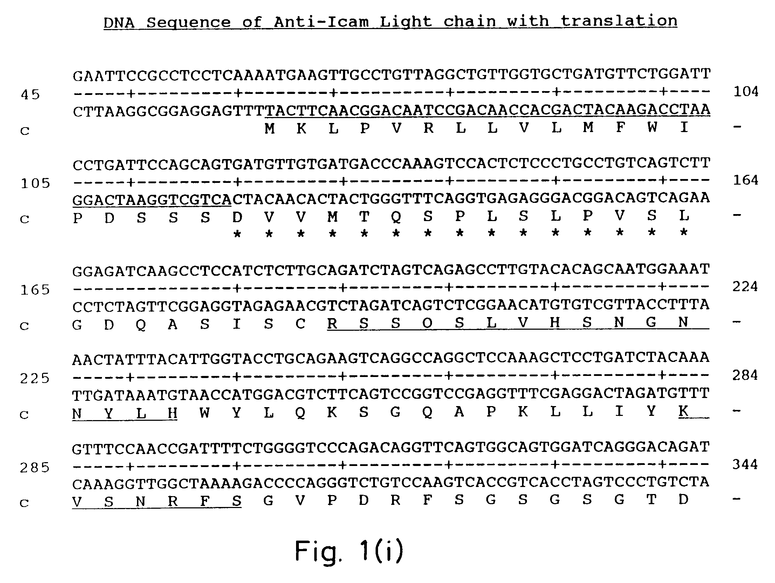

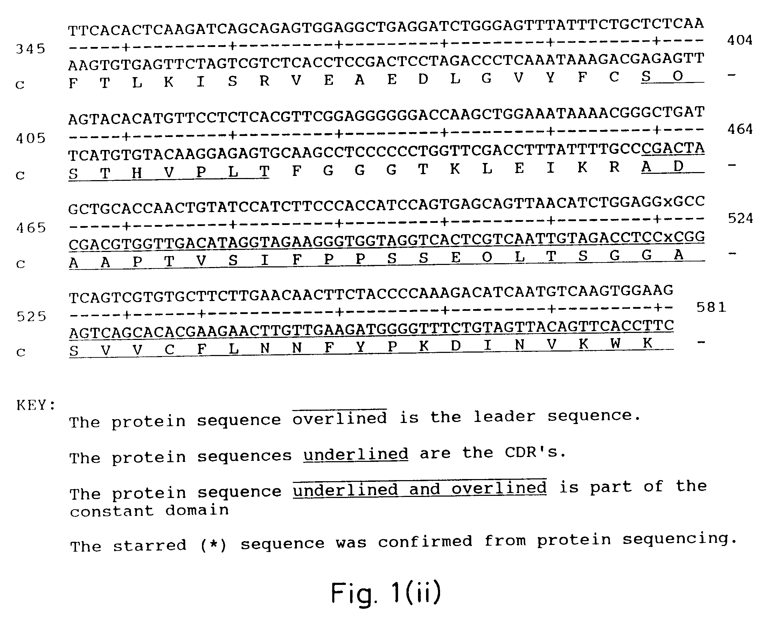

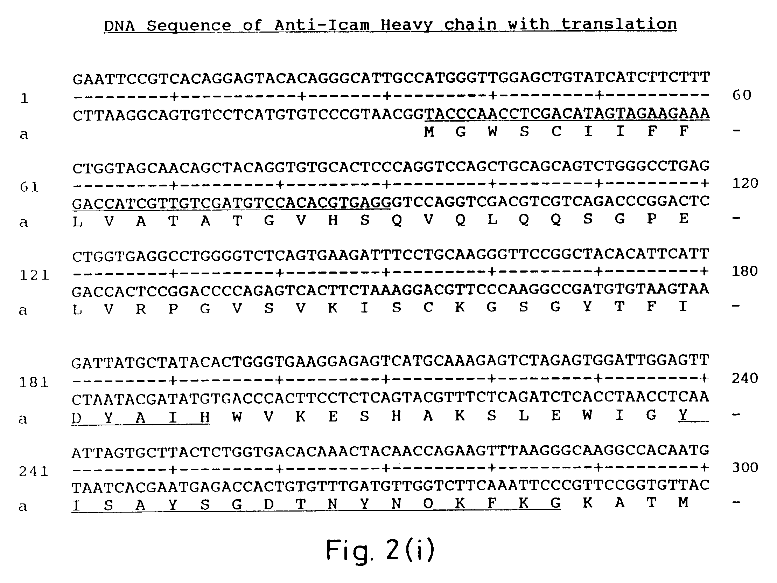

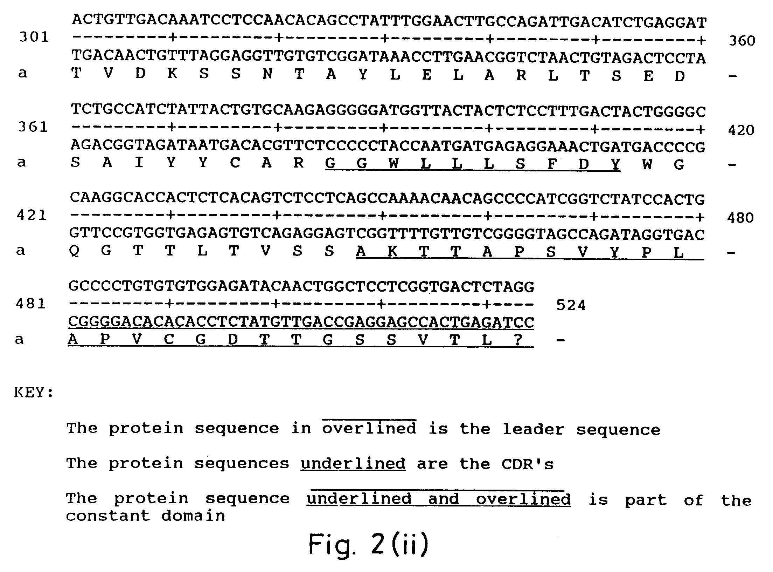

DNA sequence for the 5' untranslated regions, signal sequences, variable regions and 3' untranslated regions of full length cDNAs were obtained and are given in Figure 1 for the light chain ad Figure 2 for the heavy chain.

### 7. Construction of cDNA Expression Vectors

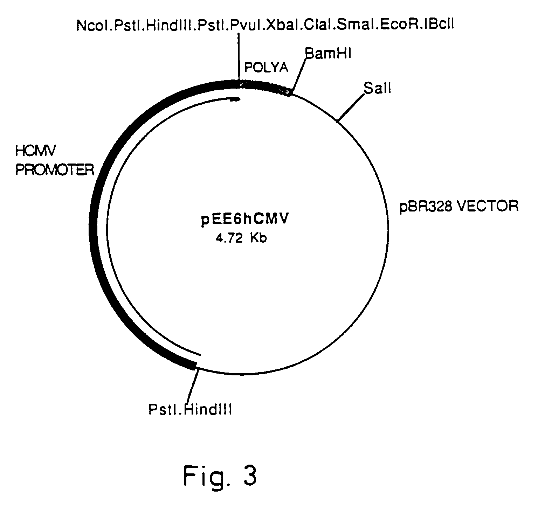

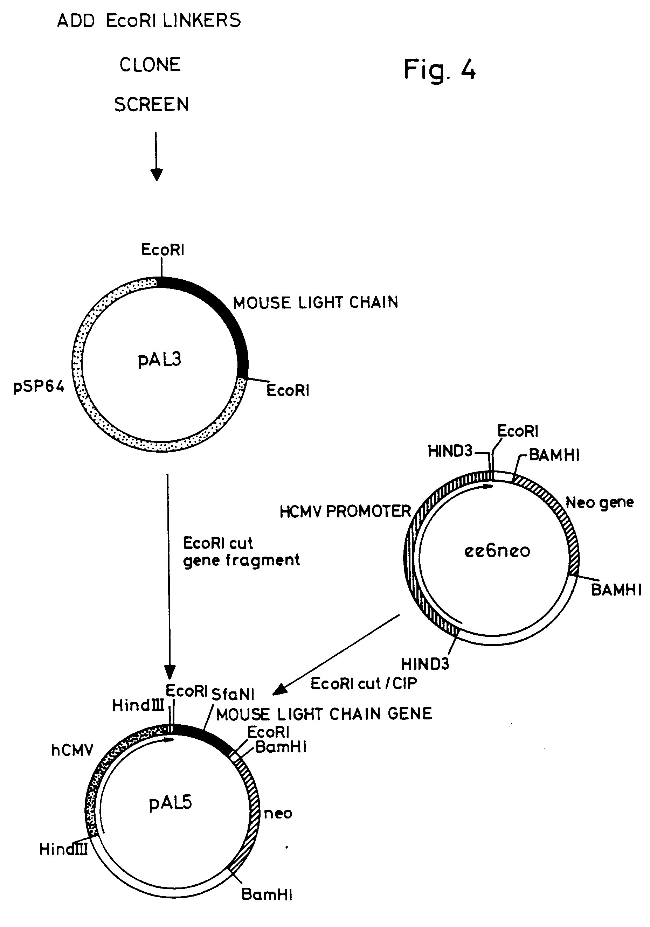

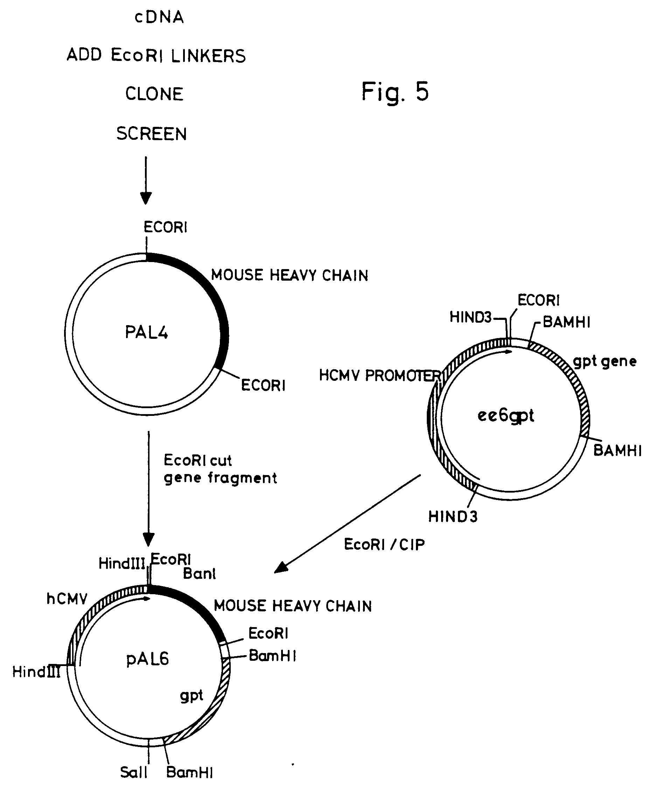

CellTech expression vectors are based on the plasmid pEE6-hCMV as shown in Figure 3 (Bebbington, C.R., Published International Patent Application WO 89/01036). A polylinker for the insertion of genes to be expressed has been introduced after the major immediate early promoter/enhance of the human Cytomegalovirus (hCMV). Marker genes for selection of the plasmid in transfected eukaryotic cells can be inserted as BamHI cassettes m the unique BamHI site of pEE6-hCMV. It is usual practice to insert the neo and gpt markers prior to insertion of the gene of interest, whereas the GS marker is inserted last because of the presence of internal EcoRI sites in the cassette. The selectable markers are expressed from the SV40 late promoter which also provides an origin of replication so that the vectors can be used for expression in the COS cell transient expression system. The mouse sequences were excised as EcoRI fragments and cloned into either EE6-hCMV-neo for the light chain (Figure 4) and into EE6-hCMV-gpt for the heavy chain (Figure 5).

### 8. Expression of cDNAs in COS cells

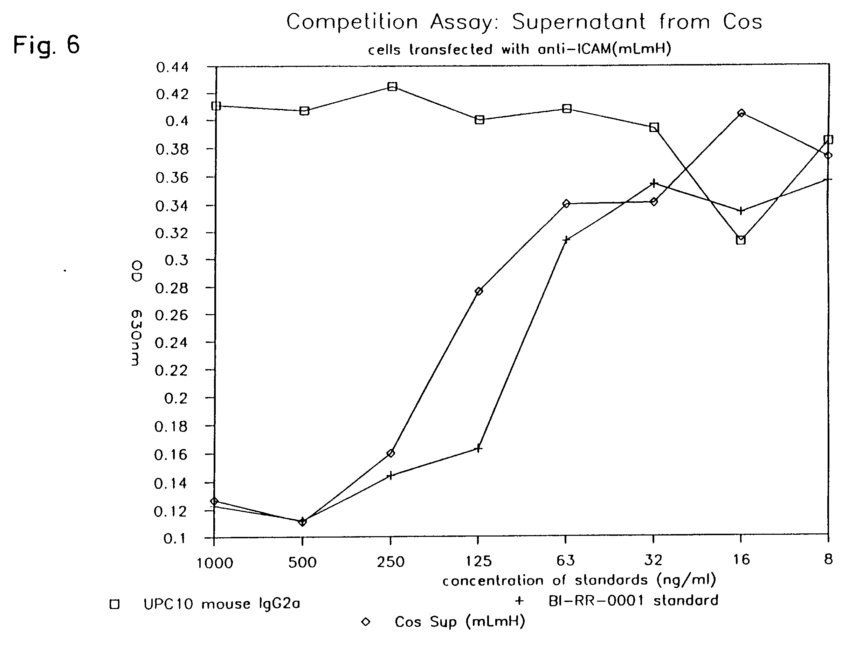

Plasmids pAL5 (Figure 4) and pAL6 (Figure 5) were co-transfected into COS cells and supernatant from the transient expression experiment was shown to contain assembled antibody which bound to JY cells (Figure 6). Metabolic labelling experiments using ³⁵S methionine showed expression and assembly of heavy and light chains.

### 9. Construction of Chimeric Genes

Construction of chimeric genes followed a previously described strategy (Whittle *et al.* 1987, (Whittle *et al., Prot. Eng. 1, 6*:499-505 (1987)). A restriction site near the 3' end of the variable domain sequence is identified and used to attach an oligonucleotide adapter which codes for the remainder of the mouse variable region and includes a suitable restriction site for attachment to the constant region of choice.

### 9.1 Light Chain Gene Construction

The mouse light chain cDNA sequence showed an SfaNI site near the 3' end of the variable region. The majority of the sequence of the variable region was isolated as a 397 bp. EcoRI-SfaNI fragment. An oligonucleotide adapter was designed to replace the remainder of the 3' region of the variable region from the SfaNI site and to include the 5' residues of the human constant region up to and including a unique NarI site which had been previously engineered into the constant region. The linker was ligated to the human C_{K} gene in NarI cut pRB32 and the SfaNI-EcoRI adapted C_{K} fragment was purified from the ligation mixture. The constant region was ligated with the EcoRI-SfaNI cut variable region DNA into an EcoRI/CIP pEE6-hCMV-neo treated vector in a three way reaction. Clones were isolated after transformation into *E. coli* and the linker and junction sequences were confirmed by DNA sequencing.

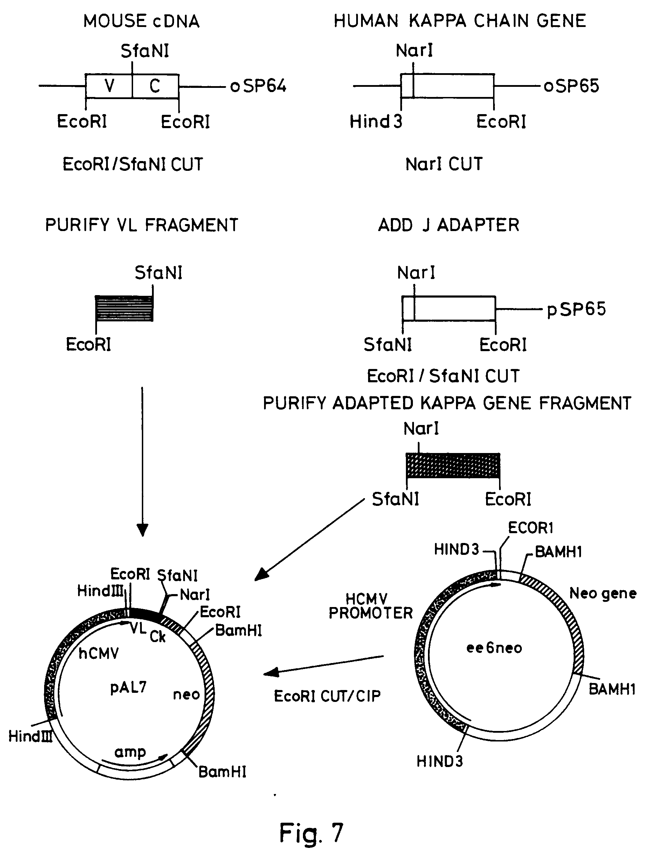

Figure 7 shows the strategy for construction of the chimeric light chain.

### 9.2 Heavy Chain Gene Construction

### 9.2.1. Choice of Heavy Chain Gene Isotype

Chimeric heavy chain genes coding for both human IgG2 and IgG4 isotypes were constructed.

### 9.2.2. Gene Construction

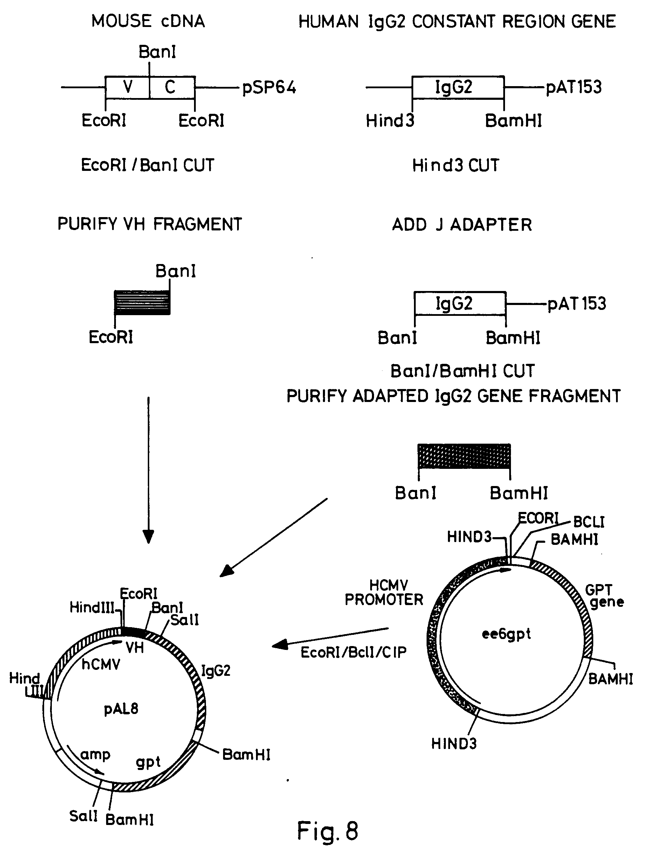

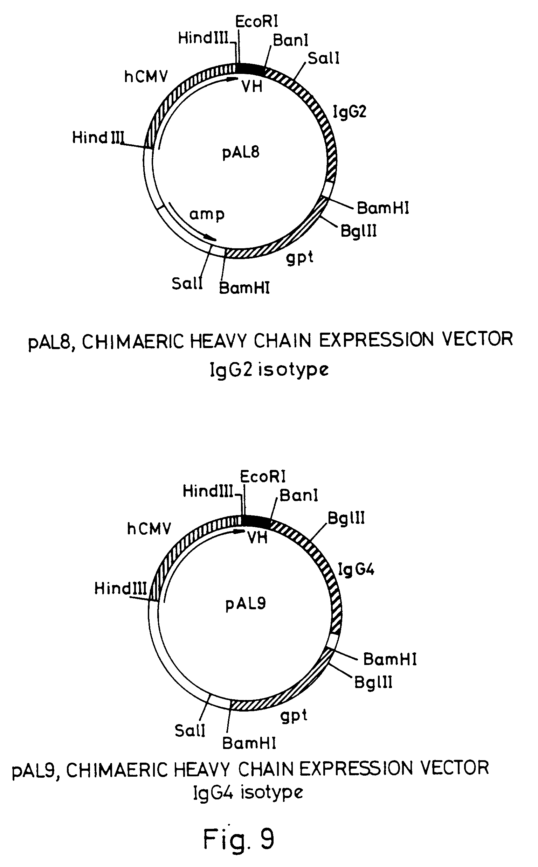

The heavy chain cDNA sequence showed a BanI site near the 3' end of the variable region. The majority of the sequence of the variable region was isolated as a 424bp EcoRI/CIP/BanI fragment. An oligonucleotide adapter was designated to replace the remainder of the 3' region of the variable region from the BanI site up to and including a unique HindIII site which had been previously engineered into the first two amino acids of the constant region. The linker was ligated to the C_{H} gene fragments in HindIII cut pRB41 and pRB21 and the BanI-BamHI adapted constant region fragments were purified from the ligation mixture. The EcoRI-BanI variable region fragment was ligated to each of the constant regions and into the expression vector (EcoRI/BciI/CIP treated pEE6-hCMV-gpt) via a three way ligation. Clones were isolated after transformation into *E. coli* HB101 and the linker and junction sequences were confirmed by DNA sequencing. Figure 8 shows the strategy for the construction of the chimeric IgG2 and IgG4 heavy chains and Figure 9 shows an outline plasmid map of pAL8 (IgG2) and pAL9 (IgG4).

### 9.2.3 IgG1 Heavy Chain Gene Construction

Plasmid pE1001 is an expression vector based on pEE6.hCMV gpt. It contains the human IgG1 constant region gene. The ApaI site which occurs at the 5th and 6th codon of the Ch1 domain is unique in this vector, as is a HindIII site 3' to the hCMV promoter. The VH region of the anti-ICAM-1 heavy chain gene along with the sequence encoding the first five residues of human CH1 was isolated from pAL9 and inserted into pE1001, previously cut with HindIII and ApaI, to give pJA200. The CH1 residues carried over from pAL9 are identical to those for IgG1 therefore no novel sequence is generated at the V-C junction.

### 10. Construction of Chimeric Expression Vectors

### 10.1 GS Separate Vectors

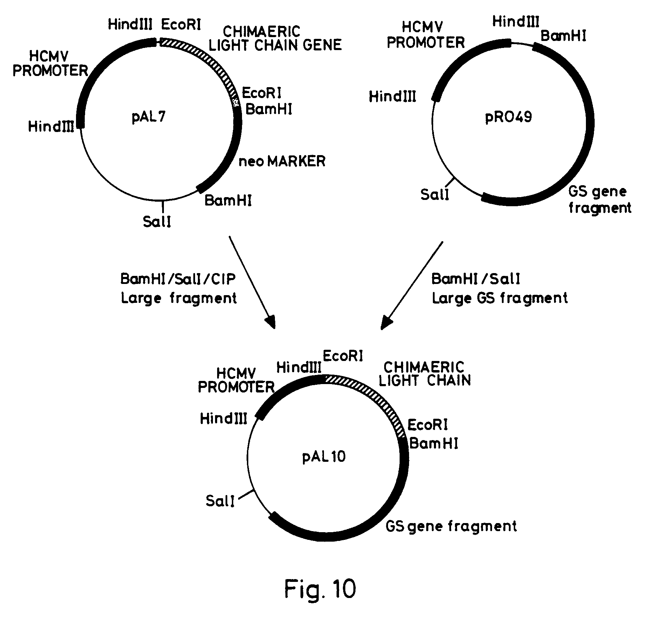

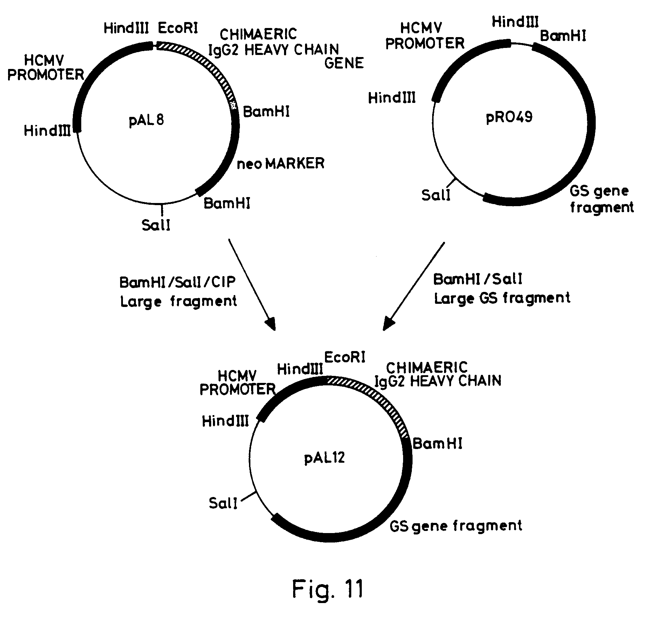

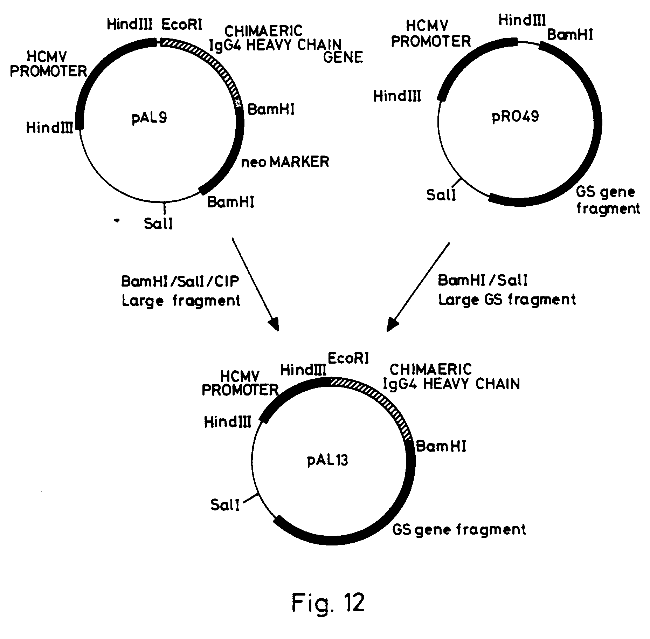

GS versions of pAL7, pAL8 and pAL9 (Figures 7, 8 and 9) were constructed by replacing the neo and gpt BamH1 cassettes with a 5.9Kbp cassette containing the GS gene capable of being expressed from the SV40 late promoter (See Figures 7, 10-12 for plasmid drawings).

### 11. Expression of Chimeric Genes

### 11.1 Expression in COS Cells

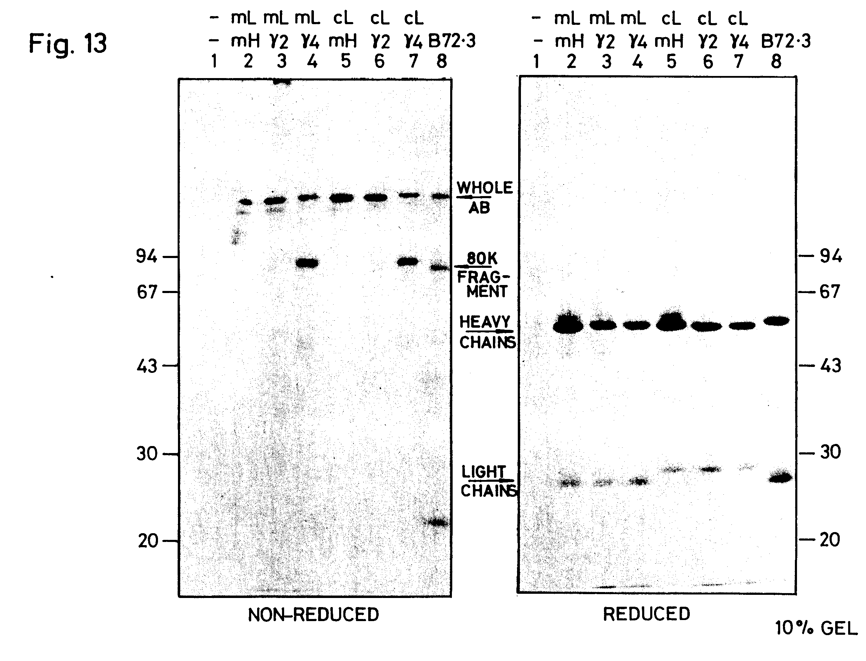

The chimeric antibody plasmid pAL7 (cL), with either pAL8 (cHIgG2), pAL9 (cHIgG4), or pJA200 (CHIgG1) was co-transfected into COS cells and supernatant from the transient expression experiment was shown to contain assembled antibody which bound to the JY human B-cell line. Metabolic antibody which bound to the JY human B-cell line. Metabolic labelling experiments using ³⁵S methionine showed expression and assembly of heavy and light chains (Figure 13). All chimeric antibodies bound well to the JY cells. However, the competition assay showed that antibody derived from the cL/cHIgG2 or the cL/cHIgG4 combination did not compete as well as the biotinylated mouse antibody for ICAM-1. The cL/cHIgG1 antibody competed better than the cL/cHIgG2 or cL/cHBIgG4 antibodies.

### 11.2 Expression in Chinese Hamster Ovary (CHO) Cells

Stable cell lines were prepared as follows: Chimeric light chain expression vectors pAL7 and pAL10 containing either the neo or GS markers were transfected into CHO-K1 cells by the CaPO₄ precipitation procedure. After growth on selective medium, positive cell lines were identified and their specific production rates measured using and ELISA format assay for detection of secreted light chain. The two cell lines secreting the highest levels of light chain (see table below) were selected ad retransfected with either pAL8 or pAL9 to introduce the IgG2 and IgG4 chimeric heavy chain genes along with the gpt marker.

| CELL LINE | SELECTABLE MARKER | SPECIFIC PRODUCTION RATE (pg./cell/day) |
|---|---|---|
| 24 | neo | 2.1 |
| 46 | neo | 0.9 |
| 25 | GS | 2.0 |
| 27 | GS | 2.3 |

Around 24 lines shown to be secreting chimeric antibody by assembly assay ELISA were taken from each transfection. Specific production rates were measured and the 8 lines with the highest specific production rates are shown below.

| CELL LINE | SPECIFIC PRODUCTION RATE (pg./Cell/day) |
|---|---|
| neo 24 G2-9 | 6.9 |
| neo 24 G2-233 | 9.0 |
| neo 24 G4-19 | 3.0 |
| neo 24 G4-22 | 4.2 |
| neo 24 G4-24 | 7.5 |
| GS 25 G2-12 | 11.7 |
| GS 25 G4-5 | 2.1 |
| GS 27 G4-8 | 2.3 |

The chimeric IgG1 heavy chain expression vector pJA200 containing the gpt marker was transfected into the chimeric light chain expressing cell line neo24 by the CaPO4 precipitation method. Specific production rates were measured, the 4 best lines are shown below.

| CELL LINE | SPECIFIC PRODUCTION RATE (pg./cell/day) |
|---|---|
| 24.1.48 | 3.75 |
| 24.2.7 | 1.2 |
| 24.1.41 | 1.3 |
| 24.2.11 | 1.75 |

### 12. Purification of Chimeric Antibody

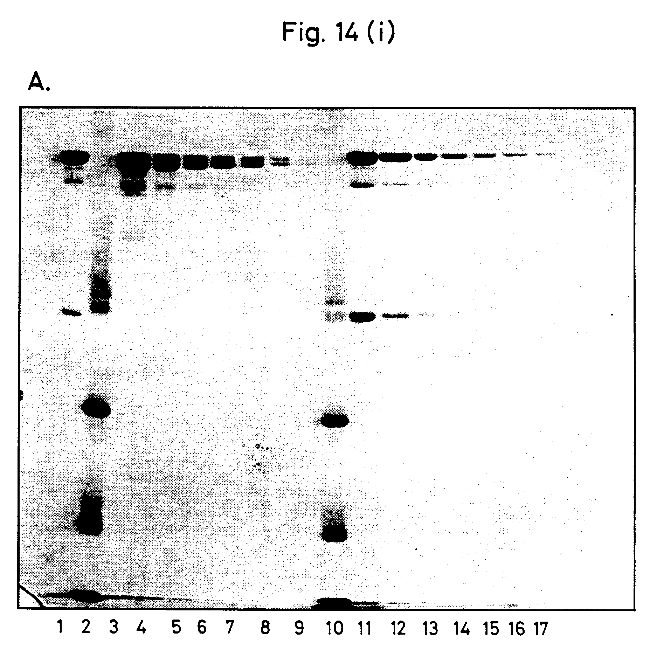

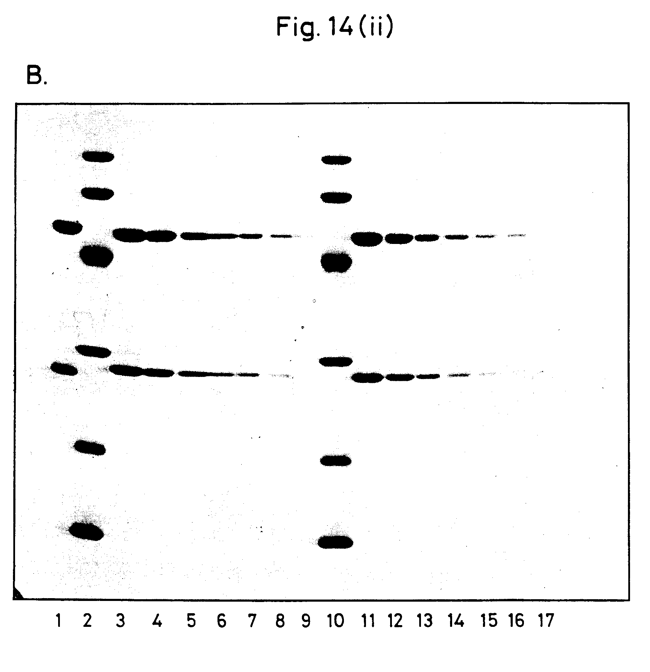

Antibody was purified from 2x1L harvests of supernatant from roller cultures for cell lines GS 25 G2-12, neo 24 G4-24 and 24.1.48, and also from 2x0.5L harvests from neo 24 G2-9 by affinity chromatography using Protein A Sepharose. Cell culture supernatants were adjusted to pH8.8 with 0.2M sodium glycinate and applied to a protein A Sepharose column which had been pre-equilibrated with glycine/glycinate buffer at pH8.8. After the samples had been loaded the column was washed with quilibration buffer. The antibody was then eluted by applying a solution with a deceasing pH gradient consisting of 0.2M disodium hydrogen phosphate ad 0.1M citric acid. Antibody containing fractions were pooled and the pH adjusted to 6.5 the samples were then dialyzed against phosphate buffered saline (PBS). Purity and correct assembly of the antibody was tested by reducing and non-reducing SDS-polyacrylamide gel electrophoresis (SDS-PAGE) (Fig. 14) and by high performance liquid chromatography (HPLC) gel filtration (Figure 15). Identity was confirmed by N-terminal amino acid sequencing and amino acid composition analysis.

### 13. Analysis of Purified Antibody

### 13.1 Result of Direct Binding and Competitive Binding Assays

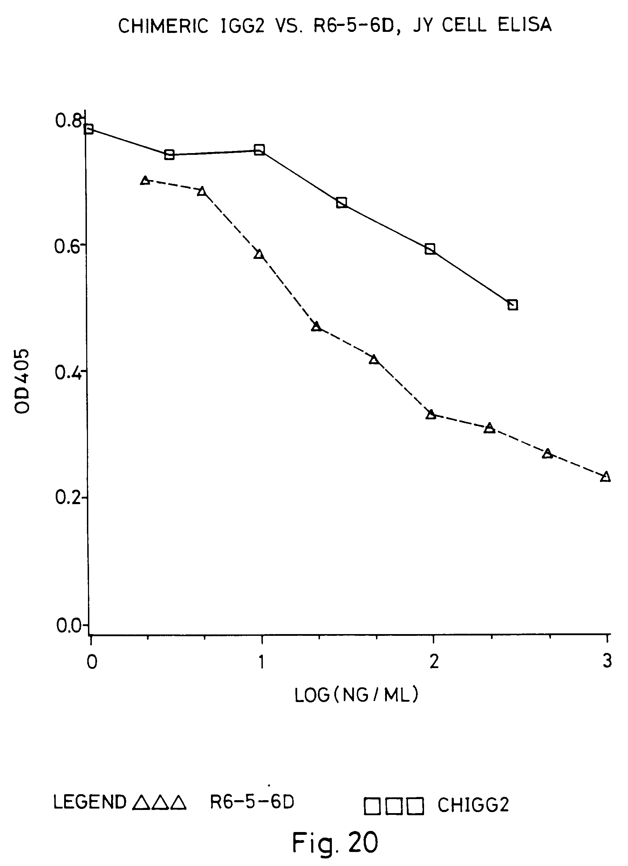

All chimeric antibodies bound well to the JY cells (Figures 16 and 17). However, in competition assays only the cL/cHIgG1 antibody competed nearly as well as the mouse antibody against the biotinylated mouse antibody for binding to ICAM (Fig. 18). The cL/cHIgG4 antibody showed about 30% of the inhibitory activity of the mouse antibody (Fig. 19) and the cL/cHIgG2 antibody showed about 10% of the inhibitory activity of the mouse antibody (Fig. 20).

These data show chimeric anti-ICAM-1 antibodies derived from R6-5-D6 have different antigen binding activities depending on isotype. The IgG1 antibody is nearly as avid as the mouse parent antibody. The IgG4 antibody as 30% of the competitive binding activity of the mouse antibody, the IgG2 has 10% relative activity. This result is unexpected as all these antibodies have identical binding sites. We attribute these differences to avidity alterations imposed on the antibodies by the differing hinge flexibility of the isotypes. Affinity measurements of the chimeric IgG4 Fab have shown that this has the same affinity as the mouse Fab thus confirming that it is the avidity that has altered in construction of the chimeric IgG4.

### 13.2 Results of Mixed Lymphocyte Reaction Assays

The MRL which is an in vitro model of transplantation was inhibited by the chimeric IgG4 and IgG1 to comparable extent as the mouse r6-5-6D antibody. This shows that the chimeric MAb will inhibit specific immunological events and can thus be used in in vivo autoimmune and transplantation settings (see Fig. 21).

### 13.3 Results of Modified Schwartzmann Reaction Assays

The primate Schwartzmann reaction was set up to test granulocyte function in vivo in the presence of an anti-ICAM-1 MAb. The chimeric IgG1 was slightly more active than the mouse MAb which in turn was slightly more active in inhibiting activated neutrophil mediated vascular leakage than the chimeric IgG4 in the primate model. This demonstrates that the chimeric anti-ICAM-1's will be effective in mitigating neutrophil mediated damage associated with reperfusion injury and other acute inflammatory disorders (see Fig. 22).

## Claims (Claims for the following Contracting State(s): AT, BE, CH, DE, DK, FR, GB, IT, LI, LU, NL, SE)

1. A humanised chimeric antibody molecule comprising heavy and/or light chain regions of the murine anti-ICAM-1 antibody R6-5-D6, producible by the hybridoma deposited at the A.T.C.C. with the accession number HB 9580, and an IgG1 human constant region, wherein said chimeric antibody binds to ICAM-1 with an avidity substantially equivalent to that of the murine R6-5-D6 antibody.

2. The humanised chimeric antibody molecule of claim 1 comprising at least one chimeric heavy chain and at least one chimeric light chain.

3. A pharmaceutical composition comprising the humanised chimeric antibody of claim 1 or 2 and a pharmaceutically acceptable carrier.

4. The pharmaceutical composition of claim 3, additionally comprising at least one other immunosuppressive agent.

5. A DNA molecule encoding the humanised chimeric antibody of claims 1 or 2.

6. The DNA molecule of claim 5 which is a vector.

7. The DNA molecule of claim 6, wherein a DNA sequence encoding the humanised chimeric antibody of claim 1 or 2 is in operative combination with a promoter sequence.

8. A host cell transformed with a DNA molecule according to any one of claims 6 or 7.

9. The use of a chimeric antibody according to any one of claims 1 or 2 in the preparation of an anti-inflammatory agent and/or an agent for treating inflammation resulting from a response of the specific defence system in a mammalian subject.

10. The use of a chimeric antibody according to any one of claims 1 or 2 in the preparation of an agent for treating inflammation resulting from a response of the non-specific defence system in a mammalian subject.

11. The use of a chimeric antibody according to any one of claims 1 or 2 in the preparation of an anti-inflammatory agent for suppressing the metastasis of a hematopoietic tumour cell, the cell requiring a functional member of the LFA-1 family for migration.

12. The use of a chimeric antibody according to any one of claims 1 or 2 in the preparation of an agent for suppressing the growth of an ICAM-1-expressing tumour cell.

13. The use of a chimeric antibody according to any one of claims 1 or 2 sufficient to suppress viral infection in the preparation of an agent for treating a viral infection in an individual.

14. The use of a chimeric antibody according to any one of claims 1 or 2 in the preparation of an agent for suppressing the infection of leukocytes with HIV, such as HIV-1.

15. The use of a chimeric antibody according to any one of claims 1 or 2 in the preparation of an anti-migration agent for suppressing the extravascular migration of a virally infected leukocyte in a patient having such a leukocyte, for example the virally infected cells being infected with HIV.

16. The use of a chimeric antibody according to any one of claims 1 or 2 in the preparation of an agent for treating asthma.

17. The use according to claim 9 or 16 wherein the inflammation is:
(a) a delayed type hypersensitivity reaction;
(b) a symptom of psoriasis;
(c) a symptom of an autoimmune disease, such as Reynaud's syndrome, autoimmune thyroiditis, EAE, multiple sclerosis, rheumatoid arthritis and/or lupus erythematosus;
(d) in response to organ transplant rejection, such as kidney transplant; and/or
(e) in response to tissue graft rejection.

18. The use according to any of claims 9, 16 or 17 which additionally comprises the use of: an antibody capable of binding to LFA-1; a functional derivative of the antibody, the functional derivative being capable of binding to LFA-1; and/or a non-immunoglobulin antagonist of LFA-1.

19. The use according to claim 10 or 16 wherein the inflammation is associated with adult respiratory distress syndrome; multiple organ injury syndrome secondary to septicaemia; multiple organ injury syndrome secondary to trauma; reperfusion injury of tissue; acute glomerulonephritis; reactive arthritis; dermatosis with acute inflammatory components; a central nervous system inflammatory disorder e.g. stroke; thermal injury; hemodialysis; leukapheresis; ulcerative colitis; Crohn's disease; necrotising enterocolitis; granulocyte transfusion associated syndrome; and/or cytokine-induced toxicity.

20. The use according to claim 13 wherein the virus is a rhinovirvs of the major serotype within the genus Picornaviridae, a group A cocksackievirus, or a Mengo virus.

21. The use according to any of claims 9 to 20 wherein the chimeric antibody is administered by enteral means, parenteral means (such as intramuscular, intravenous or subcutaneous), topical means, inhalation means or intranasal means.

22. The use according to any of claims 9 to 21 wherein the chimeric antibody is administered prophylactically and/or therapeutically.

23. The use of chimeric antibody according to any one of claims 1 or 2 for the preparation of a diagnostic agent for a method of diagnosing an ICAM-1-expressing tumour cell in a mammalian subject, the method comprising:
(a) administering to a subject a composition containing a detectably labelled chimeric antibody capable of binding to ICAM-1; and
(b) detecting any chimeric antibody bound to ICAM-1.

24. The use of chimeric antibody according to any one of claims 1 or 2 for the preparation of a diagnostic agent for a method of diagnosing inflammation in a mammalian subject, the method comprising:
(a) incubating a sample of tissue from a subject with a composition containing a detectably labelled chimeric antibody capable of binding to a cell which expresses ICAM-1; and
(b) detecting any chimeric antibody bound to the cell.

## Claims (Claims for the following Contracting State(s): ES)

1. A process for the production of an anti-ICAM-1 humanised chimeric antibody, the process comprising:
(1) producing an expression vector comprising an operon having a DNA sequence which encodes an antibody heavy or light chain wherein at least one of the CDRs of the variable domain are derived from the murine anti-ICAM-1 antibody R6-5-D6, producible by the hybridoma deposited at the A.T.C.C. with the accession number HB 9580, and the remaining immunoglobulin-derived parts of the antibody chain are derived from a human immunoglobulin, the human constant region being of the IgG1 subtype;
(2) producing an expression vector comprising an operon having a DNA sequence which encodes a complementary antibody light or heavy chain wherein at least one of the CDRs of the variable domain are derived from the murine anti-ICAM-1 antibody R6-5-D6 producible by the hybridoma deposited at the A.T.C.C. with the accession number HB 9580, and the remaining immunoglobulin-derived parts of the antibody chain are derived from a human immunoglobulin, the human constant region being of the IgG1 subtype;
(3) in place of stages 1 and 2, producing a single expression vector having all the features of the vectors in 1 and 2 which encodes both the light chain- and heavy chain-derived polypeptides;
(4) transfecting a host cell with each vector; and
(5) culturing the transfected cell line to produce the chimeric antibody, wherein said chimeric antibody binds to ICAM-1 with an avidity substantially equivalent to that of the murine R6-5-D6 antibody.

2. A process according to claim 1 wherein the antibody comprises at least one chimeric heavy chain and at least one chimeric light chain.

3. A process according to claim 1 or 2 additionally comprising attaching to the antibody an effector or reporter molecule.

4. A process for the preparation of a vector, the process comprising constructing a vector that includes a DNA sequence encoding an anti-ICAM-1 humanised chimeric antibody according to claim 1 or 2.

5. A process according to claim 4 for preparing an expression vector comprising in operative combination DNA coding for a chimeric anti-ICAM-1 light chain and a chimeric anti-ICAM-1 heavy chain.

6. A transfection method comprising transforming a host cell with a vector prepared according to any of claims 4 or 5.

7. A process for the preparation of a pharmaceutical composition the process comprising admixing a chimeric antibody prepared according to any of claims 1 to 3 with a pharmaceutically acceptable carrier.

8. A process according to claim 7 additionally comprising admixing at least one other immunosuppressive agent.

9. The use of an anti-ICAM-1 humanised chimeric antibody prepared by a method according to any one of claims 1 to 3 for the preparation of a composition for a method of diagnosing an ICAM-1-expressing tumour cell in a mammalian subject, the method comprising:
(a) administering to a subject a composition containing a detectably labelled chimeric antibody capable of binding to ICAM-1; and
(b) detecting any chimeric antibody bound to ICAM-1.

10. A method of diagnosing inflammation in a mammalian subject, the method comprising:
(a) incubating a sample of tissue from a subject with a composition containing a detectably labelled chimeric anti-ICAM-1 antibody prepared by a process according to any one of claims 1 to 3; and
(b) detecting any chimeric antibody bound to the cell.

11. The use of a chimeric anti -ICAM-1 antibody prepared by a process according to anyone of claims 1 to 3 in the preparation of an anti-inflammatory agent and/or an agent for treating inflammation resulting from a response of the specific defence system in a mammalian subject.

12. The use of a chimeric anti -ICAM-1 antibody prepared by a process according to any one of claims 1 to 3 in the preparation of an agent for treating inflammation resulting from a response of the non-specific defence system in a mammalian subject.

13. The use of a chimeric anti-ICAM-1 antibody prepared by a process according to any one of claims 1 to 3 in the preparation of an anti-inflammatory agent for suppressing the metastasis of a hematopoietic tumour cell, the cell requiring a functional member of the LFA-1 family for migration.

14. The use of a chimeric anti -ICAM-1 antibody prepared by a process according to any one of claims 1 to 3 in the preparation of an agent for suppressing the growth of an ICAM-1-expressing tumour cell.

15. The use of a chimeric anti -ICAM-1 antibody prepared by a process according to any one of claims 1 to 3 in the preparation of an agent for treating a viral infection in an individual.

16. The use of a chimeric anti -ICAM-1 antibody prepared by a process according to any one of claims 1 to 3 in the preparation of an agent for suppressing the infection of leukocytes with HIV, such as HIV-1.

17. The use of a chimeric anti -ICAM-1 antibody prepared by a process according to any one of claims 1 to 3 in the preparation of an anti-migration agent for suppressing the extravascular migration of a virally infected leukocyte in a patient having such a leukocyte, for example the virally infected cells being infected with HIV.

18. The use of a chimeric anti -ICAM-1 antibody prepared by a process according to any one of claims 1 to 3 in the preparation of an agent for treating asthma.

19. The use according to claim 11 wherein the inflammation is:
(a) a delayed type hypersensitivity reaction;
(b) a symptom of psoriasis;
(c) a symptom of an autoimmune disease, such as Reynaud's syndrome, autoimmune thyroiditis, EAE, multiple sclerosis, rheumatoid arthritis and/or lupus erythematosus;
(d) in response to organ transplant rejection, such as kidney transplant; and/or
(e) in response to tissue graft rejection.

20. The use according to any of claims 11 or 19 which additionally comprises the use of: an antibody capable of binding to LFA-1; a functional derivative of the antibody, the functional derivative being capable of binding to LFA-1; and/or a non-immunoglobulin antagonist of LFA-1.

21. The use according to claim 12 wherein the inflammation is associated with adult respiratory distress syndrome; multiple organ injury syndrome secondary to septicaemia; multiple organ injury syndrome secondary to trauma; reperfusion injury of tissue; acute glomerulonephritis; reactive arthritis; dermatosis with acute inflammatory components; a central nervous system inflammatory disorder e.g. stroke; thermal injury; hemodialysis; leukapheresis; ulcerative colitis; Crohn's disease; necrotising enterocolitis; granulocyte transfusion associated syndrome; and/or cytokine-induced toxicity.

22. The use according to claim 15 wherein the virus is a rhinovirus of the major serotype within the genus Picornaviridae, a group A cocksackievirus, or a Mengo virus.

23. The use according to any of claims 11 to 22 wherein the chimeric antibody is administered by enteral means, parenteral means (such as intramuscular, intravenous or subcutaneous), topical means, inhalation means or intranasal means.

24. The use according to any of claims 11 to 23 wherein the chimeric antibody is administered prophylactically and/or therapeutically.

## Claims (Claims for the following Contracting State(s): GR)

1. A process for the production of an anti-ICAM-1 humanised chimeric antibody, the process comprising:
(1) producing an expression vector comprising an operon having a DNA sequence which encodes an antibody heavy or light chain wherein at least one of the CDRs of the variable domain are derived from the murine anti-ICAM-1 antibody R6-5-D6, producible by the hybridoma deposited at the A.T.C.C. with the accession number HB 9580, and the remaining immunoglobulin-derived parts of the antibody chain are derived from a human immunoglobulin, the human constant region being of the IgG1 subtype;
(2) producing an expression vector comprising an operon having a DNA sequence which encodes a complementary antibody light or heavy chain wherein at least one of the CDRs of the variable domain are derived from the murine anti-ICAM-1 antibody R6-5-D6, producible by the hybridoma deposited at the A.T.C.C. with the accession number HB 9580, and the remaining immunoglobulin-derived parts of the antibody chain are derived from a human immunoglobulin, the human constant region being of the IgG1 subtype;
(3) in place of stages 1 and 2, producing a single expression vector having all the features of the vectors in 1 and 2 which encodes both the light chain- and heavy chain-derived polypeptides;
(4) transfecting a host cell with each vector; and
(5) culturing the transfected cell line to produce the chimeric antibody, wherein said chimeric antibody binds to ICAM-1 with an avidity substantially equivalent to that of the murine R6-5-D6 antibody.

2. A process according to claim 1 wherein the antibody comprises at least one chimeric heavy chain and at least one chimeric light chain.

3. A process according to claim 1 or 2 additionally comprising attaching to the antibody an effector or reporter molecule.

4. A DNA sequence encoding the heavy or light chain variable region of an anti-ICAM-1 antibody prepared by the process of any one of claims 1 to 3.

5. A vector comprising DNA according to claim 4.

6. An vector according to claim 4 or 5 which is an expression vector comprising in operative combination DNA coding for a chimeric anti-ICAM-1 light chain and a chimeric anti-ICAM-1 heavy chain.

7. A host cell transformed with a vector according to claim 5 or 6.

8. A process for the preparation of a pharmaceutical composition the process comprising admixing a chimeric antibody prepared according to any of claims 1 to 3 with a pharmaceutically acceptable carrier.

9. A process according to claim 8 additionally comprising admixing at least one other immunosuppressive agent.

10. The use of a chimeric anti -ICAM-1 antibody prepared by a process according to anyone of claims 1 to 3 in the preparation of an anti-inflammatory agent and/or an agent for treating inflammation resulting from a response of the specific defence system in a mammalian subject.

11. The use of a chimeric anti -ICAM-1 antibody prepared by a process according to any one of claims 1 to 3 in the preparation of an agent for treating inflammation resulting from a response of the non-specific defence system in a mammalian subject.

12. The use of a chimeric anti -ICAM-1 antibody prepared by a process according to any one of claims 1 to 3 in the preparation of an anti-inflammatory agent for suppressing the metastasis of a hematopoietic tumour cell, the cell requiring a functional member of the LFA-1 family for migration.

13. The use of a chimeric anti -ICAM-1 antibody prepared by a process according to any one of claims 1 to 3 in the preparation of an agent for suppressing the growth of an ICAM-1-expressing tumour cell.

14. The use of a chimeric anti -ICAM-1 antibody prepared by a process according to any one of claims 1 to 3 in the preparation of an agent for treating a viral infection in an individual.

15. The use of a chimeric anti -ICAM-1 antibody prepared by a process according to any one of claims 1 to 3 in the preparation of an agent for suppressing the infection of leukocytes with HIV, such as HIV-1.

16. The use of a chimeric anti -ICAM-1 antibody prepared by a process according to any one of claims 1 to 3 in the preparation of an anti-migration agent for suppressing the extravascular migration of a virally infected leukocyte in a patient having such a leukocyte, for example the virally infected cells being infected with HIV.

17. The use of a chimeric anti -ICAM-1 antibody prepared by a process according to any one of claims 1 to 3 in the preparation of an agent for treating asthma.

18. The use according to claim 10 wherein the inflammation is:
(a) a delayed type hypersensitivity reaction;
(b) a symptom of psoriasis;
(c) a symptom of an autoimmune disease, such as Reynaud's syndrome, autoimmune thyroiditis, EAE, multiple sclerosis, rheumatoid arthritis and/or lupus erythematosus;
(d) in response to organ transplant rejection, such as kidney transplant; and/or
(e) in response to tissue graft rejection.

19. The use according to any of claims 10 or 19 which additionally comprises the use of: an antibody capable of binding to LFA-1; a functional derivative of the antibody, the functional derivative being capable of binding to LFA-1; and/or a non-immunoglobulin antagonist of LFA-1.

20. The use according to claim 11 wherein the inflammation is associated with adult respiratory distress syndrome; multiple organ injury syndrome secondary to septicaemia; multiple organ injury syndrome secondary to trauma; reperfusion injury of tissue; acute glomerulonephritis; reactive arthritis; dermatosis with acute inflammatory components; a central nervous system inflammatory disorder e.g. stroke; thermal injury; hemodialysis; leukapheresis; ulcerative colitis; Crohn's disease; necrotising enterocolitis; granulocyte transfusion associated syndrome; and/or cytokine-induced toxicity.

21. The use according to claim 14 wherein the virus is a rhinovirus of the major serotype within the genus Picornaviridae, a group A cocksackievirus, or a Mengo virus.

22. The use according to any of claims 10 to 21 wherein the chimeric antibody is administered by enteral means, parenteral means (such as intramuscular, intravenous or subcutaneous), topical means, inhalation means or intranasal means.

23. The use according to any of claims 10 to 22 wherein the chimeric antibody is administered prophylactically and/or therapeutically.

24. The use of an anti-ICAM-1 humanised chimeric antibody prepared by a method according to any one of claims 1 to 3 for the preparation of a composition for a method of diagnosing an ICAM-1-expressing tumour cell in a mammalian subject, the method comprising:
(a) administering to a subject a composition containing a detectably labelled chimeric antibody capable of binding to ICAM-1; and
(b) detecting any chimeric antibody bound to ICAM-1.

25. A method of diagnosing inflammation in a mammalian subject, the method comprising:
(a) incubating a sample of tissue from a subject with a composition containing a detectably labelled chimeric anti-ICAM-1 antibody prepared by a process according to any one of claims 1 to 3; and
(b) detecting any chimeric antibody bound to the cell.

## Patentansprüche (Patentansprüche für folgende(n) Vertragsstaat(en): AT, BE, CH, DE, DK, FR, GB, IT, LI, LU, NL, SE)

1. Humanisiertes chimäres Antikörper-Molekül, das schwer- und/oder leichtkettige Bereiche des murinen Anti-ICAM-1-Antikörpers R6-5-D6, welcher von dem beim A.T.C.C. unter der Zugangsnummer HB 9580 hinterlegten Hybridom produzierbar ist, sowie einen konstanten Human-IgG1-Bereich umfasst, wobei der genannte Antikörper eine Neigung zur Bindung an ICAM-1 aufweist, welche derjenigen des murinen R6-5-D6-Antikörpers äquivalent ist.

2. Humanisiertes chimäres Antikörper-Molekül nach Anspruch 1, welches mindestens eine chimäre schwere Kette und mindestens eine chimäre leichte Kette umfasst.

3. Arzneimittelzusammensetzung, welche den humanisierten chimären Antikörper nach Anspruch 1 oder 2 und einen pharmazeutisch annehmbaren Träger umfasst.

4. Arzneimittelzusammensetzung nach Anspruch 3, welche zudem mindestens ein anderes Immunsuppressionsmittel umfasst.

5. DNA-Molekül, das für den humanisierten chimären Antikörper nach Anspruch 1 oder 2 codiert.

6. DNA-Molekül nach Anspruch 5, welches ein Vektor ist.

7. DNA-Molekül nach Anspruch 6, bei welchem eine DNA-Sequenz, die für den humanisierten chimären Antikörper nach Anspruch 1 oder 2 codiert, in funktioneller Kombination mit einer Promotor-Sequenz vorliegt.

8. Wirtszelle, welche mit einem DNA-Molekül nach einem der Ansprüche 6 oder 7 transformiert ist.

9. Verwendung eines chimären Antikörpers nach einem der Ansprüche 1 oder 2 bei der Herstellung eines Entzündungshemmers und/oder eines Mittels zur Behandlung einer Entzündung, die sich aus einer Antwort des spezifischen Abwehrsystems in einem Säuger-Subjekt ergibt.

10. Verwendung eines chimären Antikörpers nach einem der Ansprüche 1 oder 2 bei der Herstellung eines Mittels zur Behandlung einer Entzündung, die sich aus einer Antwort des nicht-spezifischen Abwehrsystems in einem Säuger-Subjekt ergibt.

11. Verwendung eines chimären Antikörpers nach einem der Ansprüche 1 oder 2 bei der Herstellung eines Entzündungshemmers zur Unterdrückung der Metastasierung einer hämatopoetischen Tumorzelle, wobei zur Migration der Zelle ein funktionelles Mitglied der LFA-1-Familie erforderlich ist.

12. Verwendung eines chimären Antikörpers nach einem der Ansprüche 1 oder 2 bei der Herstellung eines Wachstumshemmers für eine ICAM-1 exprimierende Tumorzelle.

13. Verwendung eines chimären Antikörpers nach einem der Ansprüche 1 oder 2, der genügt, um eine virale Infektion zu unterdrücken, bei der Herstellung eines Mittels zur Behandlung einer viralen Infektion in einem Individuum.

14. Verwendung eines chimären Antikörpers nach einem der Ansprüche 1 oder 2 bei der Herstellung eines Mittels zur Unterdrückung der Infektion von Leukozyten durch HIV, wie beispielsweise HIV-1.

15. Verwendung eines chimären Antikörpers nach einem der Ansprüche 1 oder 2 bei der Herstellung eines Anti-Migrations-Mittels zur Unterdrückung der extravaskularen Migration eines viral infizierten Leukozyten bei einem Patienten, der einen solchen Leukozyten aufweist, wenn beispielsweise die viral infizierten Zellen mit HIV infiziert sind.

16. Verwendung eines chimären Antikörpers nach einem der Ansprüche 1 oder 2 bei der Herstellung eines Mittels zur Behandlung von Asthma.

17. Verwendung nach Anspruch 9 oder 16, bei welcher die Entzündung:
(a) eine Überempfindlichkeitsreaktion vom verzögerten Typus ist;
(b) ein Symptom von Psoriasis ist;
(c) ein Symptom von Autoimmun-Erkrankung wie das Reynaud-Syndrom, Autoimmun-Schilddrüsenentzündung, EAE, Multiple Sclerose, rheumatoider Arthritis und/oder Lupus erythematodes ist;
(d) in Antwort auf die Abstossung eines Organtransplantats wie beispielsweise eines Nierentransplantats erfolgt; und/oder
(e) in Antwort auf die Abstossung eines Gewebetransplantats erfolgt.

18. Verwendung nach einem der Ansprüche 9, 16 oder 17 unter zusätzlicher Verwendung eines zur Bindung an LFA-1 fähigen Antikörpers; eines funktionellen Derivats des Antikörpers, wobei das funktionelle Derivat fähig ist, an LFA-1 zu binden; und/oder eines nicht-immunoglobulinartigen kompetitiven Antagonisten von LFA-1.

19. Verwendung nach Anspruch 10 oder 16, bei welcher die Entzündung assoziiert ist mit dem Erwachsenen-Atemnot-Syndrom; dem sekundär zur Septikämie auftretenden Multiplen-Organschaden-Syndrom; dem sekundär zum Trauma auftretenden Multiplen-Organschaden-Syndrom; Reperfusions-Gewebeschaden; akuter Glomerulonephritis; reaktiver Arthritis; Dermatosis mit Komponenten akuter Entzündung; entzündungsbedingter Störung des zentralen Nervensystems wie beispielsweise einem Schlaganfall; thermischem Schaden; Hämodialyse; Leukapheresis; Colitis ulcerosa; der Crohn-Krankheit; nekrotisierender Enterocolitis; dem der Granulozyten-Transfusion assoziierten Syndrom; und/oder der von Zytokin induzierter Toxizität.

20. Verwendung nach Anspruch 13, bei welcher das Virus ein Rhinovirus des Haupt-Serotypus innerhalb des Genus Picornaviridae, ein Cocksackievirus der Gruppe A oder ein Mengo-Virus ist.

21. Verwendung nach einem der Ansprüche 9 bis 20, bei welcher der chimäre Antikörper mit enteralen Mitteln, parenteralen Mitteln (wie beispielsweise intramuskulär, intravenös oder subkutan), topischen Mitteln, Inhalationsmitteln oder intranasalen Mitteln verabreicht wird.

22. Verwendung nach einem der Ansprüche 9 bis 21, bei welcher der chimäre Antikörper prophylaktisch und/oder therapeutisch verabreicht wird.

23. Verwendung eines chimären Antikörpers nach einem der Ansprüche 1 oder 2 bei der Herstellung eines Diagnosemittels für ein Verfahren zur Diagnose einer ICAM-1 exprimierenden Tumorzelle bei einem Säuger-Subjekt, wobei das Verfahren umfasst, dass man:
(a) einem Subjekt eine Zusammensetzung verabreicht, die einen detektierbar markierten, zur Bindung an ICAM-1 fähigen chimären Antikörper enthält; und
(b) jeden an ICAM-1 gebundenen chimären Antikörper detektiert.

24. Verwendung eines chimären Antikörpers nach einem der Ansprüche 1 oder 2 bei der Herstellung eines Diagnosemittels für ein Verfahren zur Diagnose von Entzündung bei einem Säuger-Subjekt, wobei das Verfahren umfasst:
(a) Inkubation einer Gewebeprobe eines Subjekts mit einer Zusammensetzung, die einen detektierbar markierten chimären Antikörper enthält, der zur Bindung an eine ICAM-1 exprimierende Zelle fähig ist, und
(b) Detektion eines jeden an die Zelle gebundenen chimären Antikörpers.

## Patentansprüche (Patentansprüche für folgende(n) Vertragsstaat(en): ES)

1. Verfahren zur Herstellung eines humanisierten chimären Anti-ICAM-1-Antikörpers, wobei dieses Verfahren umfasst:
(1) Produktion eines Expressionsvektors, welches ein Operon umfasst, das eine DNA-Sequenz aufweist, die für eine schwere oder leichte Antikörper-Kette codiert, bei der zumindest einer der CD-Rezeptoren des variablen Bereiches vom murinen Anti-ICAM-1-Antikörpers R6-5-D6 abgeleitet ist, welcher von dem beim A.T.C.C. unter der Zugangsnummer HB 9580 hinterlegten Hybridom produzierbar ist, und die übrigen von Immunoglobulin abgeleiteten Teile der Antikörper-Kette von einem humanen Immunoglobulin abgeleitet sind, wobei der humane konstante Bereich vom Sub-Typus IgG1 ist;
(2) Produktion eines Expressionsvektors, welches ein Operon umfasst, das eine DNA-Sequenz aufweist, die für eine komplementäre schwere oder leichte Antikörper-Kette codiert, bei der zumindest einer der CD-Rezeptoren des variablen Bereiches vom murinen Anti-ICAM-1-Antikörpers R6-5-D6 abgeleitet ist, welcher von dem beim A.T.C.C. unter der Zugangsnummer HB 9580 hinterlegten Hybridom produzierbar ist, und die übrigen von Immunoglobulin abgeleiteten Teile der Antikörper-Kette von einem humanen Immunoglobulin abgeleitet sind, wobei der humane konstante Bereich vom Sub-Typus IgG1 ist;
(3) Anstelle der Schritte 1 und 2, Produktion eines einzelnen Expressionsvektors, der alle Eigenschaften der Vektoren der Schritte 1 und 2 aufweist und sowohl für die von den leichten Ketten als auch für die von den schweren Ketten abgeleiteten Polypeptide codiert;
(4) Transfizierung einer Wirtszelle mit jedem der Vektoren, und
(5) Kultivierung des transfizierten Zellstammes zur Produktion des chimären Antikörpers, wobei der genannte chimäre Antikörper eine Neigung zur Bindung an ICAM-1 aufweist, welche derjenigen des murinen R6-5-D6-Antikörpers äquivalent ist.

2. Verfahren nach Anspruch 1, bei welchem der Antikörper mindestens eine chimäre schwere Kette und mindestens eine chimäre leichte Kette umfasst.

3. Verfahren nach Anspruch 1 oder 2, zudem umfassend, dass ein Effektor- oder Reporter-Molekül an den Antikörper gebunden wird.

4. Verfahren zur Herstellung eines Vektors, wobei das Verfahren die Konstruktion eines Vektors umfasst, welches eine DNA-Sequenz einschliesst, die für einen humanisierten chimären Anti-ICAM-1-Antikörper nach Anspruch 1 oder 2 codiert.

5. Verfahren nach Anspruch 4 zur Herstellung eines Expressionsvektors, der in wirkfähiger Kombination eine für eine chimäre Anti-ICAM-1 leichte Kette codierende DNA und eine für eine chimäre Anti-ICAM-1 schwere Kette codierende DNA umfasst.

6. Transfektionsverfahren, umfassend die Transformation einer Wirtszelle mit einem nach einem der Ansprüche 4 oder 5 hergestellten Vektor.

7. Verfahren zur Herstellung einer Arzneimittelzusammensetzung, wobei das Verfahren umfasst, dass einem nach einem der Ansprüche 1 bis 3 hergestellten chimären Antikörper ein pharmazeutisch annehmbarer Träger beigemischt wird.

8. Verfahren nach Anspruch 7, zudem umfassend, dass mindestens ein anderes Immunsuppressionsmittel beigemischt wird.

9. Verwendung eines gemäss einem Verfahren nach einem der Ansprüche 1 bis 3 hergestellten humanisierten chimären Anti-ICAM-1-Antikörpers bei der Herstellung einer Zusammensetzung für ein Verfahren zur Diagnose einer ICAM-1 exprimierenden Tumorzelle bei einem Säuger-Subjekt, wobei das Verfahren umfasst, dass man:
(a) einem Subjekt eine Zusammensetzung verabreicht, die einen detektierbar markierten, zur Bindung an ICAM-1 fähigen chimären Antikörper enthält; und
(b) jeden an ICAM-1 gebundenen chimären Antikörper detektiert.

10. Verfahren zur Diagnose von Entzündung bei einem Säuger-Subjekt, wobei das Verfahren umfasst:
(a) Inkubation einer Gewebeprobe eines Subjekts mit einer Zusammensetzung, die einen gemäss einem Verfahren nach einem der Ansprüche 1 bis 3 hergestellten detektierbar markierten chimären Anti-ICAM-1-Antikörper enthält; und
(b) Detektion eines jeden an die Zelle gebundenen chimären Antikörpers.

11. Verwendung eines gemäss einem Verfahren nach einem der Ansprüche 1 bis 3 hergestellten chimären Anti-ICAM-1-Antikörpers bei der Herstellung eines Entzündungshemmers und/oder eines Mittels zur Behandlung einer Entzündung, die sich aus einer Antwort des spezifischen Abwehrsystems in einem Säuger-Subjekt ergibt.

12. Verwendung eines gemäss einem Verfahren nach einem der Ansprüche 1 bis 3 hergestellten chimären Anti-ICAM-1-Antikörpers bei der Herstellung eines Mittels zur Behandlung einer Entzündung, die sich aus einer Antwort des nicht-spezifischen Abwehrsystems in einem Säuger-Subjekt ergibt.

13. Verwendung eines gemäss einem Verfahren nach einem der Ansprüche 1 bis 3 hergestellten chimären Anti-ICAM-1-Antikörpers bei der Herstellung eines Entzündungshemmers zur Unterdrückung der Metastasierung einer hämatopoetischen Tumorzelle, wobei zur Migration der Zelle ein funktionelles Mitglied der LFA-1-Familie erforderlich ist.

14. Verwendung eines gemäss einem Verfahren nach einem der Ansprüche 1 bis 3 hergestellten chimären Anti-ICAM-1-Antikörpers bei der Herstellung eines Wachstumshemmers für eine ICAM-1 exprimierende Tumorzelle.

15. Verwendung eines gemäss einem Verfahren nach einem der Ansprüche 1 bis 3 hergestellten chimären Anti-ICAM-1-Antikörpers bei der Herstellung eines Mittels zur Behandlung einer viralen Infektion in einem Individuum.

16. Verwendung eines gemäss einem Verfahren nach einem der Ansprüche 1 bis 3 hergestellten chimären Anti-ICAM-1-Antikörpers bei der Herstellung eines Mittels zur Unterdrückung der Infektion von Leukozyten durch HIV, wie beispielsweise HIV-1.

17. Verwendung eines gemäss einem Verfahren nach einem der Ansprüche 1 bis 3 hergestellten chimären Anti-ICAM-1-Antikörpers bei der Herstellung eines Anti-Migrations-Mittels zur Unterdrückung der extravaskularen Migration eines viral infizierten Leukozyten bei einem Patienten, der einen solchen Leukozyten aufweist, wenn beispielsweise die viral infizierten Zellen mit HIV infiziert sind.

18. Verwendung eines gemäss einem Verfahren nach einem der Ansprüche 1 bis 3 hergestellten chimären Anti-ICAM-1-Antikörpers bei der Herstellung eines Mittels zur Behandlung von Asthma.

19. Verwendung nach Anspruch 11, bei welcher die Entzündung:
(a) eine Überempfindlichkeitsreaktion vom verzögerten Typus ist;
(b) ein Symptom von Psoriasis ist;
(c) ein Symptom von Autoimmun-Erkrankung wie das Reynaud-Syndrom, Autoimmun-Schilddrüsenentzündung, EAE, Multiple Sclerose, rheumatoider Arthrisis und/oder Lupus erythematodes ist;
(d) in Antwort auf die Abstossung eines Organtransplantats wie beispielsweise eines Nierentransplantats erfolgt; und/oder
(e) in Antwort auf die Abstossung eines Gewebetransplantats erfolgt.

20. Verwendung nach einem der Ansprüche 11 oder 19 unter zusätzlicher Verwendung eines zur Bindung an LFA-1 fähigen Antikörpers; eines funktionellen Derivats des Antikörpers, wobei das funktionelle Derivat fähig ist, an LFA-1 zu binden; und/oder eines nicht-immunoglobulinartigen Antagonisten von LFA-1.

21. Verwendung nach Anspruch 12, bei welcher die Entzündung assoziiert ist mit dem Erwachsenen-Atemnot-Syndrom; dem sekundär zur Septikämie auftretenden Multiplen-Organschaden-Syndrom; dem sekundär zum Trauma auftretenden Multiplen-Organschaden-Syndrom; Reperfusions-Gewebeschaden; akuter Glomerulonephritis; reaktiver Arthritis; Dermatosis mit Komponenten akuter Entzündung; entzündungsbedingter Störung des zentralen Nervensystems wie beispielsweise einem Schlaganfall; thermischem Schaden; Hämodialyse; Leukapheresis; Colitis ulcerosa; der Crohn-Krankheit; nekrotisierender Enterocolitis; dem der Granulozyten-Transfusion assoziierten Syndrom; und/oder der von Zytokin induzierter Toxizität.

22. Verwendung nach Anspruch 15, bei welcher das Virus ein Rhinovirus des Haupt-Serotypus innerhalb des Genus Picornaviridae, ein Cocksackievirus der Gruppe A oder ein Mengo-Virus ist.

23. Verwendung nach einem der Ansprüche 11 bis 22, bei welcher der chimäre Antikörper mit enteralen Mitteln, parenteralen Mitteln (wie beispielsweise intramuskulär, intravenös oder subkutan), topischen Mitteln, Inhalationsmitteln oder intranasalen Mitteln verabreicht wird.

24. Verwendung nach einem der Ansprüche 11 bis 23, bei welcher der chimäre Antikörper prophylaktisch und/oder therapeutisch verabreicht wird.

## Patentansprüche (Patentansprüche für folgende(n) Vertragsstaat(en): GR)

1. Verfahren zur Herstellung eines humanisierten chimären Anti-ICAM-1-Antikörpers, wobei dieses Verfahren umfasst:
(1) Produktion eines Expressionsvektors, welches ein Operon umfasst, das eine DNA-Sequenz aufweist, die für eine schwere oder leichte Antikörper-Kette codiert, bei der zumindest einer der CD-Rezeptoren des variablen Bereiches vom murinen Anti-ICAM-1-Antikörpers R6-5-D6 abgeleitet ist, welcher von dem beim A.T.C.C. unter der Zugangsnummer HB 9580 hinterlegten Hybridom produzierbar ist, und die übrigen von Immunoglobulin abgeleiteten Teile der Antikörper-Kette von einem humanen Immunoglobulin abgeleitet sind, wobei der humane konstante Bereich vom Sub-Typus IgG1 ist;
(2) Produktion eines Expressionsvektors, welches ein Operon umfasst, das eine DNA-Sequenz aufweist, die für eine komplementäre schwere oder leichte Antikörper-Kette codiert, bei der zumindest einer der CD-Rezeptoren des variablen Bereiches vom murinen Anti-ICAM-1-Antikörpers R6-5-D6 abgeleitet ist, welcher von dem beim A.T.C.C. unter der Zugangsnummer HB 9580 hinterlegten Hybridom produzierbar ist, und die übrigen von Immunoglobulin abgeleiteten Teile der Antikörper-Kette von einem humanen Immunoglobulin abgeleitet sind, wobei der humane konstante Bereich vom Sub-Typus IgG1 ist;
(3) Anstelle der Schritte 1 und 2, Produktion eines einzelnen Expressionsvektors, der alle Eigenschaften der Vektoren der Schritte 1 und 2 aufweist und sowohl für die von den leichten Ketten als auch für die von den schweren Ketten abgeleiteten Polypeptide codiert;
(4) Transfizierung einer Wirtszelle mit jedem der Vektoren, und
(5) Kultivierung des transfizierten Zellstammes zur Produktion des chimären Antikörpers, wobei der genannte chimäre Antikörper eine Neigung zur Bindung an ICAM-1 aufweist, welche derjenigen des murinen R6-5-D6-Antikörpers äquivalent ist.

2. Verfahren nach Anspruch 1, bei welchem der Antikörper mindestens eine chimäre schwere Kette und mindestens eine chimäre leichte Kette umfasst.

3. Verfahren nach Anspruch 1 oder 2, zudem umfassend, dass ein Effektor- oder Reporter-Molekül an den Antikörper gebunden wird.

4. DNA-Sequenz, die für einen schwer- und/oder leichtkettigen variablen Bereich eines gemäss einem Verfahren nach einem der Ansprüche 1 bis 3 hergestellten Anti-ICAM-1-Antikörpers codiert.

5. Vektor, welcher eine DNA nach Anspruch 4 umfasst.

6. Vektor nach einem der Ansprüche 4 oder 5, welcher ein Expressionsvektor ist, der in wirkfähiger Kombination eine für eine chimäre Anti-ICAM-1 leichte Kette codierende DNA und eine für eine chimäre Anti-ICAM-1 schwere Kette codierende DNA umfasst.

7. Wirtszelle, die mit einem Vektor nach einem der Ansprüche 5 oder 6 transformiert ist.

8. Verfahren zur Herstellung einer Arzneimittelzusammensetzung, wobei das Verfahren umfasst, dass einem nach einem der Ansprüche 1 bis 3 hergestellten chimären Antikörper ein pharmazeutisch annehmbarer Träger beigemischt wird.

9. Verfahren nach Anspruch 8, zudem umfassend, dass mindestens ein anderes Immunsuppressionsmittel beigemischt wird.

10. Verwendung eines gemäss einem Verfahren nach einem der Ansprüche 1 bis 3 hergestellten chimären Anti-ICAM-1-Antikörpers bei der Herstellung eines Entzündungshemmers und/oder eines Mittels zur Behandlung einer Entzündung, die sich aus einer Antwort des spezifischen Abwehrsystems in einem Säuger-Subjekt ergibt.

11. Verwendung eines gemäss einem Verfahren nach einem der Ansprüche 1 bis 3 hergestellten chimären Anti-ICAM-1-Antikörpers bei der Herstellung eines Mittels zur Behandlung einer Entzündung, die sich aus einer Antwort des nicht-spezifischen Abwehrsystems in einem Säuger-Subjekt ergibt.

12. Verwendung eines gemäss einem Verfahren nach einem der Ansprüche 1 bis 3 hergestellten chimären Anti-ICAM-1-Antikörpers bei der Herstellung eines Entzündungshemmers zur Unterdrückung der Metastasierung einer hämatopoetischen Tumorzelle, wobei zur Migration der Zelle ein funktionelles Mitglied der LFA-1-Familie erforderlich ist.

13. Verwendung eines gemäss einem Verfahren nach einem der Ansprüche 1 bis 3 hergestellten chimären Anti-ICAM-1-Antikörpers bei der Herstellung eines Wachstumshemmers für eine ICAM-1 exprimierende Tumorzelle.

14. Verwendung eines gemäss einem Verfahren nach einem der Ansprüche 1 bis 3 hergestellten chimären Anti-ICAM-1-Antikörpers bei der Herstellung eines Mittels zur Behandlung einer viralen Infektion in einem Individuum.

15. Verwendung eines gemäss einem Verfahren nach einem der Ansprüche 1 bis 3 hergestellten chimären Anti-ICAM-1-Antikörpers bei der Herstellung eines Mittels zur Unterdrückung der Infektion von Leukozyten durch HIV, wie beispielsweise HIV-1.

16. Verwendung eines gemäss einem Verfahren nach einem der Ansprüche 1 bis 3 hergestellten chimären Anti-ICAM-1-Antikörpers bei der Herstellung eines Anti-Migrations-Mittels zur Unterdrückung der extravaskularen Migration eines viral infizierten Leukozyten bei einem Patienten, der einen solchen Leukozyten aufweist, wenn beispielsweise die viral infizierten Zellen mit HIV infiziert sind.

17. Verwendung eines gemäss einem Verfahren nach einem der Ansprüche 1 bis 3 hergestellten chimären Anti-ICAM-1-Antikörpers bei der Herstellung eines Mittels zur Behandlung von Asthma.

18. Verwendung nach Anspruch 10, bei welcher die Entzündung:
(a) eine Überempfindlichkeitsreaktion vom verzögerten Typus ist;
(b) ein Symptom von Psoriasis ist;
(c) ein Symptom von Autoimmun-Erkrankung wie das Reynaud-Syndrom, Autoimmun-Schilddrüsenentzündung, EAE, Multiple Sclerose, rheumatoider Arthritis und/oder Lupus erythematodes ist;
(d) in Antwort auf die Abstossung eines Organtransplantats wie beispielsweise eines Nierentransplantats erfolgt; und/oder
(e) in Antwort auf die Abstossung eines Gewebestransplantats erfolgt.

19. Verwendung nach einem der Ansprüche 10 oder 19 unter zusätzlicher Verwendung eines zur Bindung an LFA-1 fähigen Antikörpers; eines funktionellen Derivats des Antikörpers, wobei das funktionelle Derivat fähig ist, an LFA-1 zu binden; und/oder eines nicht-immunoglobulinartigen Antagonisten von LFA-1.

20. Verwendung nach Anspruch 11, bei welcher die Entzündung assoziiert ist mit dem Erwachzenen-Atemnot-Syndrom; dem sekundär zur Septikämie auftretenden Multiplen-Organschaden-Syndrom; dem sekundär zum Traume auftretenden Multiplen-Organschaden-Syndrom; Reperfusions-Gewebeschaden; akuter Glomerulonephritis; reaktiver Arthritis; Dermatosis mit Komponenten akuter Entzündung; entzündungsbedingter Störung des zentralen Nervensystems wie beispielsweise einem Schlaganfall; thermischem Schaden; Hämodialyse; Leukapheresis; Colitis ulcerosa; der Crohn-Krankheit; nekrotisierender Enterocolitis; dem der Granulozyten-Transfusion assoziierten Syndrom; und/oder der von Zytokin induzierter Toxizität.

21. Verwendung nach Anspruch 14, bei welcher das Virus ein Rhinovirus des Haupt-Serotypus innerhalb des Genus Picornaviridae, ein Cocksackievirus der Gruppe A oder ein Mengo-Virus ist.

22. Verwendung nach einem der Ansprüche 10 bis 21, bei welcher der chimäre Antikörper mit enteralen Mitteln, parenteralen Mitteln (wie beispielsweise intramuskulär, intravenös oder subkutan), topischen Mitteln, Inhalationsmitteln oder intranasalen Mitteln verabreicht wird.

23. Verwendung nach einem der Ansprüche 10 bis 22, bei welcher der chimäre Antikörper prophylaktisch und/oder therapeutisch verabreicht wird.

24. Verwendung eines gemäss einem Verfahren nach einem der Ansprüche 1 bis 3 hergestellten humanisierten chimären Anti-ICAM-1-Antikörpers bei der Herstellung einer Zusammensetzung für ein Verfahren zur Diagnose einer ICAM-1 exprimierenden Tumorzelle bei einem Säuger-Subjekt, wobei des Verfahren umfasst, dass man:
(a) einem Subjekt eine Zusammensetzung vorabreicht, die einen detektierbar markierten, zur Bindung an ICAM-1 fähigen chimären Antikörper enthält; und
(b) jeden an ICAM-1 gebundenen chimären Antikörper detektiert.

25. Verfahren zur Diagnose von Entzündung bei einem Säuger-Subjekt, wobei das Verfahren umfasst:
(a) Inkubation einer Gewebeprobe eines Subjekts mit einer Zusammensetzung, die einen gemäss einem Verfahren nach einem der Ansprüche 1 bis 3 hergestellten detektierbar markierten chimären Anti-ICAM-1-Antikörper enthält; und
(b) Detektion eines jeden an die Zelle gebundenen chimären Antikörpers.

## Revendications (Revendications pour l'(les) Etat(s) contractant(s) suivant(s): AT, BE, CH, DE, DK, FR, GB, IT, LI, LU, NL, SE)

1. Molécule d'anticorps chimérique humanisé comprenant des régions de chaînes lourdes et/ou légères de l'anticorps anti-ICAM-1 R6-5-D6, qui peut être produite par l'hybridome déposé auprès de la A.T.C.C. sous le numéro d'ordre HB 9580, et une région constante humaine d'IgG1, où ledit anticorps chimérique se lie à ICAM-1 avec une avidité sensiblement équivalente à celle de l'anticorps R6-5-D6 murin.

2. Molécule d'anticorps chimérique humanisé selon la revendication 1 comprenant au moins une chaîne lourde chimérique et au moins une chaîne légère chimérique.

3. Composition pharmaceutique comprenant l'anticorps chimérique humanisé selon la revendication 1 ou 2 et un support pharmaceutiquement acceptable.

4. Composition pharmaceutique selon la revendication 3 comprenant en outre au moins un autre agent immunosuppresseur.

5. Molécule d'ADN codant l'anticorps chimérique humanisé selon la revendication 1 ou 2.

6. Molécule d'ADN selon la revendication 5 qui est un vecteur.

7. Molécule d'ADN selon la revendication 6 où une séquence d'ADN codant l'anticorps chimérique humanisé selon la revendication 1 ou 2 est en combinaison active avec une séquence promotrice.

8. Cellule hôte transformée avec une molécule d'ADN selon l'une quelconque des revendications 6 et 7.

9. Utilisation d'un anticorps chimérique selon l'une quelconque des revendications 1 et 2 dans la préparation d'un agent anti-inflammatoire et/ou d'un agent pour traiter une inflammation résultant d'une réponse du système de défense spécifique chez un sujet mammifère.

10. Utilisation d'un anticorps chimérique selon l'une quelconque des revendications 1 et 2 dans la préparation d'un agent pour traiter une inflammation résultant d'une réponse du système de défense non spécifique chez un sujet mammifère.

11. Utilisation d'un anticorps chimérique selon l'une quelconque des revendications 1 et 2 dans la préparation d'un agent anti-inflammatoire pour supprimer la métastase d'une cellule tumorale hématopoïétique, la cellule nécessitant un membre fonctionnel de la famille LFA-1 pour la migration.

12. Utilisation d'un anticorps chimérique selon l'une quelconque des revendications 1 et 2 dans la préparation d'un agent pour supprimer la croissance d'une cellule tumorale exprimant ICAM-1.

13. Utilisation d'un anticorps chimérique selon l'une quelconque des revendications 1 et 2 qui suffit pour supprimer une infection virale dans la préparation d'un agent pour traiter une infection virale chez un individu.

14. Utilisation d'un anticorps chimérique selon l'une quelconque des revendications 1 et 2 dans la préparation d'un agent pour supprimer l'infection de leucocytes avec VIH tel que VIH-1.

15. Utilisation d'un anticorps chimérique selon l'une quelconque des revendications 1 et 2 dans la préparation d'un agent anti-migration pour supprimer la migration extravasculaire d'un leucocyte infecté viralement chez un patient ayant un tel leucocyte, par exemple les cellules infectées viralement étant infectées par VIH.

16. Utilisation d'un anticorps chimérique selon l'une quelconque des revendications 1 et 2 dans la préparation d'un agent pour traiter l'asthme.

17. Utilisation selon la revendication 9 ou 16 où l'inflammation est:
(a) une réaction d'hypersensibilité de type retardé;
(b) un symptôme de psoriasis;
(c) un symptôme d'une maladie auto-immune, comme le syndrome de Reynaud, la tyroïdite auto-immune, EAE, la sclérose en plaques, la polyarthrite rhumatoïde et/ou le lupus érythémateux;
(d) en réponse à un rejet de transplantation d'organe, comme une transplantation de rein; et/ou
(e) en réponse à un rejet de greffe de tissu.

18. Utilisation selon l'une quelconque des revendications 9, 16 et 17 qui comprend en outre l'utilisation d'un anticorps capable de se lier à LFA-1, d'un dérivé fonctionnel de l'anticorps, le dérivé fonctionnel étant capable de se lier à LFA-1, et/ou d'un antagoniste non immunoglobulinique de LFA-1.

19. Utilisation selon la revendication 10 ou 16 où l'inflammation est associée au syndrome de détresse respiratoire aiguë de l'adulte, au syndrome de lésions d'organes multiples secondaire à une septicémie, au syndrome de lésions d'organes multiples secondaire à un traumatisme, à une lésion tissulaire de reperfusion, à la glomérulonéphrite aiguë, à l'arthrite réactive, à une dermatose à composantes inflammatoires aiguës, à un trouble inflammatoire du système nerveux central, par exemple ictus, à une lésion thermique, à l'hémodialyse, à la leucophérèse, à la colite ulcéreuse, a la maladie de Crohn, à l'entérocolite nécrosante, au syndrome associé à une transfusion de granulocytes, et/ou à une toxicité induite par des cytokines.

20. Utilisation selon la revendication 13 où le virus est un rhinovirus du sérotype majeur du genre des Picornaviridae, un virus Cocksackie du groupe A ou un virus Mengo.

21. Utilisation selon l'une quelconque des revendications 9 à 20 où l'anticorps chimérique est administré par des moyens entéraux, des moyens parentéraux (tels qu'intramusculaires, intraveineux ou sous-cutanés), des moyens topiques, des moyens par inhalation ou des moyens intranasaux.

22. Utilisation selon l'une quelconque des revendications 9 à 21 où l'anticorps chimérique est administré de manière prophylactique et/ou thérapeutique.

23. Utilisation d'un anticorps chimérique selon l'une quelconque des revendications 1 et 2 pour la préparation d'un agent de diagnostic pour un procédé de diagnostic d'une cellule tumorale exprimant ICAM-1 chez un sujet mammifère, le procédé comprenant:
(a) l'administration à un sujet d'une composition contenant un anticorps chimérique marqué de manière détectable capable de se lier à ICAM-1, et
(b) la détection de tout anticorps chimérique lié à ICAM-1.

24. Utilisation d'un anticorps chimérique selon l'une quelconque des revendications 1 et 2 pour la préparation d'un agent de diagnostic pour un procédé de diagnostic d'une inflammation chez un sujet mammifère, le procédé comprenant:
(a) l'incubation d'un échantillon de tissu provenant d'un sujet avec une composition contenant un anticorps chimérique marqué de manière détectable capable de se lier à une cellule qui exprime ICAM-1, et
(b) la détection de tout anticorps chimérique lié à la cellule.

## Revendications (Revendications pour l'(les) Etat(s) contractant(s) suivant(s): ES)

1. Procédé pour la production d'un anticorps chimérique humanisé anti-ICAM-1, le procédé comprenant:
(1) la production d'un vecteur d'expression comprenant un opéron ayant une séquence d'ADN qui code une chaîne lourde ou légère d'anticorps où au moins l'une des CDR du domaine variable est dérivée de l'anticorps anti-ICAM-1 R6-5-D6 murin, qui peut être produit par l'hybridome déposé auprès de la A.T.C.C. sous le numéro d'ordre HB 9580, et les parties dérivées d'immunoglobuline restantes de la chaîne d'anticorps sont dérivées d'une immunoglobuline humaine, la région constante humaine étant du sous-type IgG1;
(2) la production d'un vecteur d'expression comprenant un opéron ayant une séquence d'ADN qui code une chaîne légère ou lourde d'anticorps complémentaire où au moins l'une des CDR du domaine variable est dérivée de l'anticorps anti-ICAM-1 R6-5-D6 murin, qui peut être produit par l'hybridome déposé auprès de la A.T.C.C. sous le numéro d'ordre HB 9580, et les parties dérivées d'immunoglobuline restantes de la chaîne d'anticorps sont dérivées d'une immunoglobuline humaine, la région constante humaine étant du sous-type IgG1;
(3) à la place des étapes 1 et 2, la production d'un vecteur d'expression unique ayant toutes les caractéristiques des vecteurs 1 et 2 qui code les polypeptides dérivés des chaînes légères et des chaînes lourdes;
(4) la transfection d'une cellule hôte avec chaque vecteur; et
(5) la culture de la lignée cellulaire transfectée pour produire l'anticorps chimérique, où ledit anticorps chimérique se lie à ICAM-1 avec une avidité sensiblement équivalente à celle de l'anticorps R6-5-D6 murin.

2. Procédé selon la revendication 1 dans lequel l'anticorps comprend au moins une chaîne lourde chimérique et au moins une chaîne légère chimérique.

3. Procédé selon la revendication 1 ou 2 comprenant en outre la fixation à l'anticorps d'une molécule effectrice ou reporter.

4. Procédé pour la préparation d'un vecteur, le procédé comprenant la construction d'un vecteur qui comprend une séquence d'ADN codant un anticorps chimérique humanisé anti-ICAM-1 selon la revendication 1 ou 2.

5. Procédé selon la revendication 4 pour préparer un vecteur d'expression comprenant en combinaison active un ADN codant une chaîne légère anti-ICAM-1 chimérique et une chaîne lourde anti-ICAM-1 chimérique.

6. Procédé de transfection comprenant la transformation d'une cellule hôte avec un vecteur préparé selon l'une quelconque des revendications 4 et 5.

7. Procédé pour la préparation d'une composition pharmaceutique, le procédé comprenant le mélange d'un anticorps chimérique préparé selon l'une quelconque des revendications 1 à 3 avec un support pharmaceutiquement acceptable.

8. Procédé selon la revendication 7 comprenant en outre le mélange d'au moins un autre agent immunosuppresseur.

9. Utilisation d'un anticorps chimérique humanisé anti-ICAM-1 préparé par un procédé selon l'une quelconque des revendications 1 à 3 pour la préparation d'une composition pour un procédé de diagnostic d'une cellule tumorale exprimant ICAM-1 chez un sujet mammifère, le procédé comprenant:
(a) l'administration à un sujet d'une composition contenant un anticorps chimérique marqué de manière détectable capable de se lier à ICAM-1; et
(b) la détection de tout anticorps chimérique lié à ICAM-1.

10. Procédé de diagnostic d'une inflammation chez un sujet mammifère, le procédé comprenant:
(a) l'incubation d'un échantillon de tissu provenant d'un sujet avec une composition contenant un anticorps chimérique anti-ICAM-1 marqué de manière détectable préparé par un procédé selon l'une quelconque des revendications 1 à 3, et
(b) la détection de tout anticorps chimérique lié à la cellule.

11. Utilisation d'un anticorps chimérique anti-ICAM-1 préparé par un procédé selon l'une quelconque des revendications 1 à 3 dans la préparation d'un agent anti-inflammatoire et/ou d'un agent pour traiter une inflammation résultant d'une réponse du système de défense spécifique chez un sujet mammifère.

12. Utilisation d'un anticorps chimérique anti-ICAM-1 préparé par un procédé selon l'une quelconque des revendications 1 à 3 dans la préparation d'un agent pour traiter une inflammation résultant d'une réponse du système de défense non spécifique chez un sujet mammifère.

13. Utilisation d'un anticorps chimérique anti-ICAM-1 préparé par un procédé selon l'une quelconque des revendications 1 à 3 dans la préparation d'un agent anti-inflammatoire pour supprimer la métastase d'une cellule tumorale hématopoïétique, la cellule nécessitant un membre fonctionnel de la famille LFA-1 pour la migration.

14. Utilisation d'un anticorps chimérique anti-ICAM-1 préparé par un procédé selon l'une quelconque des revendications 1 à 3 dans la préparation d'un agent pour supprimer la croissance d'une cellule tumorale exprimant ICAM-1.

15. Utilisation d'un anticorps chimérique anti-ICAM-1 préparé par un procédé selon l'une quelconque des revendications 1 à 3 dans la préparation d'un agent pour traiter une infection virale chez un individu.

16. Utilisation d'un anticorps chimérique anti-ICAM-1 préparé par un procédé selon l'une quelconque des revendications 1 à 3 dans la préparation d'un agent pour supprimer l'infection de leucocytes par VIH tel que VIH-1.

17. Utilisation d'un anticorps chimérique anti-ICAM-1 préparé par un procédé selon l'une quelconque des revendications 1 à 3 dans la préparation d'un agent anti-migration pour supprimer la migration extravasculaire d'un leucocyte infecté viralement chez un patient ayant un tel leucocyte, par exemple les cellules infectées viralement étant infectées par VIH.

18. Utilisation d'un anticorps chimérique anti-ICAM-1 préparé par un procédé selon l'une quelconque des revendications 1 à 3 dans la préparation d'un agent pour traiter l'asthme.

19. Utilisation selon la revendication 11 où l'inflammation est:
(a) une réaction d'hypersensibilité de type retardé;
(b) un symptôme de psoriasis;
(c) un symptôme d'une maladie auto-immune, comme le syndrome de Reynaud, la tyroïdite auto-immune, EAE, la sclérose en plaques, la polyarthrite rhumatoïde et/ou le lupus érythémateux;
(d) en réponse à un rejet de transplantation d'organe, comme une transplantation de rein; et/ou
(e) en réponse à un rejet de greffe de tissu.

20. Utilisation selon l'une quelconque des revendications 11 et 19 qui comprend en outre l'utilisation d'un anticorps capable de se lier à LFA-1, d'un dérivé fonctionnel de l'anticorps, le dérivé fonctionnel étant capable de se lier à LFA-1, et/ou d'un antagoniste non immunoglobulinique de LFA-1.

21. Utilisation selon la revendication 12 où l'inflammation est associée au syndrome de détresse respiratoire aiguë de l'adulte, au syndrome de lésions d'organes multiples secondaire à une septicémie, au syndrome de lésions d'organes multiples secondaire à un traumatisme, à une lésion tissulaire de reperfusion, à la glomérulonéphrite aiguë, à l'arthrite réactive, à une dermatose à composantes inflammatoires aiguës, à un trouble inflammatoire du système nerveux central, par exemple ictus, à une lésion thermique, à l'hémodialyse, à la leucophérèse, à la colite ulcéreuse, à la maladie de Crohn, à l'entérocolite nécrosante, au syndrome associé à une transfusion de granulocytes, et/ou à une toxicité induite par des cytokines.

22. Utilisation selon la revendication 15 où le virus est un rhinovirus du sérotype majeur du genre des Picornaviridae, un virus Cocksackie du groupe A ou un virus Mengo.

23. Utilisation selon l'une quelconque des revendications 11 à 22 où l'anticorps chimérique est administré par des moyens entéraux, des moyens parentéraux (tels qu'intramusculaires, intraveineux ou sous-cutanés), des moyens topiques, des moyens par inhalation ou des moyens intranasaux.

24. Utilisation selon l'une quelconque des revendications 11 à 23 où l'anticorps chimérique est administré de manière prophylactique et/ou thérapeutique.

## Revendications (Revendications pour l'(les) Etat(s) contractant(s) suivant(s): GR)

1. Procédé pour la production d'un anticorps chimérique humanisé anti-ICAM-1, le procédé comprenant:
(1) la production d'un vecteur d'expression comprenant un opéron ayant une séquence d'ADN qui code une chaîne lourde ou légère d'anticorps où au moins l'une des CDR du domaine variable est dérivée de l'anticorps anti-ICAM-1 R6-5-D6 murin, qui peut être produit par l'hybridome déposé à la A.T.C.C. sous le numéro d'ordre HB 9580, et les parties dérivées d'immunoglobuline restantes de la chaîne d'anticorps sont dérivées d'une immunoglobuline humaine, la région constante humaine étant du sous-type IgG1;
(2) la production d'un vecteur d'expression comprenant un opéron ayant une séquence d'ADN qui code une chaîne légère ou lourde d'anticorps complémentaire où au moins l'une des CDR du domaine variable est dérivée de l'anticorps anti-ICAM-1 R6-5-D6 murin, qui peut être produit par l'hybridome déposé auprès de la A.T.C.C. sous le numéro d'ordre HB 9580, et les parties dérivées d'immunoglobuline restantes de la chaîne d'anticorps sont dérivées d'une immunoglobuline humaine, la region constante humaine étant du sous-type IgG1;
(3) à la place des étapes 1 et 2, la production d'un vecteur d'expression unique ayant toutes les caractéristiques des vecteurs 1 et 2 qui code les polypeptides dérivés des chaînes légères et des chaînes lourdes;
(4) la transfection d'une cellule hôte avec chaque vecteur; et
(5) la culture de la lignée cellulaire transfectée pour produire l'anticorps chimérique, où ledit anticorps chimérique se lie à ICAM-1 avec une avidité sensiblement équivalente à celle de l'anticorps R6-5-D6 murin.

2. Procédé selon la revendication 1 dans lequel l'anticorps comprend au moins une chaîne lourde chimérique et au moins une chaîne légère chimérique.

3. Procédé selon la revendication 1 ou 2 comprenant en outre la fixation à l'anticorps d'une molécule effectrice ou reporter.

4. Séquence d'ADN codant la région variable de chaîne lourde ou légère d'un anticorps anti-ICAM-1 préparé par le procédé selon l'une quelconque des revendication 1 à 3.

5. Vecteur comprenant un ADN selon la revendication 4.

6. Vecteur selon la revendication 4 ou 5 qui est un vecteur d'expression comprenant en combinaison active un ADN codant une chaîne légère anti-ICAM-1 chimérique et une chaîne lourde anti-ICAM-1 chimérique.

7. Cellule hôte transformée avec un vecteur selon la revendication 5 ou 6.

8. Procédé pour la préparation d'une composition pharmaceutique, le procédé comprenant le mélange d'un anticorps chimérique préparé selon l'une quelconque des revendications 1 à 3 avec un support pharmaceutiquement acceptable.

9. Procédé selon la revendication 8 comprenant en outre le mélange d'au moins un autre agent immunosuppresseur.

10. Utilisation d'un anticorps chimérique anti-ICAM-1 préparé par un procédé selon l'une quelconque des revendications 1 à 3 dans la préparation d'un agent anti-inflammatoire et/ou d'un agent pour traiter une inflammation résultant d'une réponse du système de défense spécifique chez un sujet mammifère.

11. Utilisation d'un anticorps chimérique anti-ICAM-1 préparé par un procédé selon l'une quelconque des revendications 1 à 3 dans la préparation d'un agent pour traiter une inflammation résultant d'une réponse du système de défense non spécifique chez un sujet mammifère.

12. Utilisation d'un anticorps chimérique anti-ICAM-1 préparé par un procédé selon l'une quelconque des revendications 1 à 3 dans la préparation d'un agent anti-inflammatoire pour supprimer la métastase d'une cellule tumorale hématopoïétique, la cellule nécessitant un membre fonctionnel de la famille LFA-1 pour la migration.

13. Utilisation d'un anticorps chimérique anti-ICAM-1 préparé par un procédé selon l'une quelconque des revendications 1 à 3 dans la préparation d'un agent pour supprimer la croissance d'une cellule tumorale exprimant ICAM-1.

14. Utilisation d'un anticorps chimérique anti-ICAM-1 préparé par un procédé selon l'une quelconque des revendications 1 à 3 dans la préparation d'un agent pour traiter une infection virale chez un individu.

15. Utilisation d'un anticorps chimérique anti-ICAM-1 préparé par un procédé selon l'une quelconque des revendications 1 à 3 dans la préparation d'un agent pour supprimer l'infection de leucocytes par VIH tel que VIH-1.

16. Utilisation d'un anticorps chimérique anti-ICAM-1 préparé par un procédé selon l'une quelconque des revendications 1 à 3 dans la préparation d'un agent anti-migration pour supprimer la migration extravasculaire d'un leucocyte infecté viralement chez un patient ayant un tel leucocyte, par exemple les cellules infectées viralement étant infectées par VIH.

17. Utilisation d'un anticorps chimérique anti-ICAM-1 préparé par un procédé selon l'une quelconque des revendications 1 à 3 dans la préparation d'un agent pour traiter l'asthme.

18. Utilisation selon la revendication 10 où l'inflammation est:
(a) une réaction d'hypersensibilité de type retardé;
(b) un symptôme de psoriasis;
(c) un symptôme d'une maladie auto-immune, comme le syndrome de Reynaud, la tyroïdite auto-immune, EAE, la sclérose en plaques, la polyarthrite rhumatoïde et/ou le lupus érythémateux;
(d) en réponse à un rejet de transplantation d'organe, comme une transplantation de rein; et/ou
(e) en réponse à un rejet de greffe de tissu.

19. Utilisation selon l'une quelconque des revendications 10 et 19 qui comprend en outre l'utilisation d'un anticorps capable de se lier à LFA-1, d'un dérivé fonctionnel de l'anticorps, le dérivé fonctionnel étant capable de se lier à LFA-1, et/ou d'un antagoniste non immunoglobulinique de LFA-1.

20. Utilisation selon la revendication 11 où l'inflammation est associée au syndrome de détresse respiratoire aiguë de l'adulte, au syndrome de lésions d'organes multiples secondaire à une septicémie, au syndrome de lésions d'organes multiples secondaire à un traumatisme, à une lésion tissulaire de reperfusion, à la glomérulonéphrite aiguë, à l'arthrite réactive, à une dermatose à composantes inflammatoires aiguës, à un trouble inflammatoire du système nerveux central, par exemple ictus, à une lésion thermique, à l'hémodialyse, à la leucophérèse, à la colite ulcéreuse, à la maladie de Crohn, à l'entérocolite nécrosante, au syndrome associé à une transfusion de granulocytes, et/ou à une toxicité induite par des cytokines.

21. Utilisation selon la revendication 14 où le virus est un rhinovirus du sérotype majeur du genre des Picornaviridae, un virus Cocksackie du groupe A ou un virus Mengo.

22. Utilisation selon l'une quelconque des revendications 10 à 21 où l'anticorps chimérique est administré par des moyens entéraux, des moyens parentéraux (tels qu'intramusculaires, intraveineux ou sous-cutanés), des moyens topiques, des moyens par inhalation ou des moyens intranasaux.

23. Utilisation selon l'une quelconque des revendications 10 à 22 où l'anticorps chimérique est administré de manière prophylactique et/ou thérapeutique.

24. Utilisation d'un anticorps chimérique humanisé anti-ICAM-1 préparé par un procédé selon l'une quelconque des revendications 1 à 3 pour la préparation d'une composition pour un procédé de diagnostic d'une cellule tumorale exprimant ICAM-1 chez un sujet mammifère, le procédé comprenant:
(a) l'administration à un sujet d'une composition contenant un anticorps chimérique marqué de manière détectable capable de se lier à ICAM-1; et
(b) la détection de tout anticorps chimérique lié à ICAM-1.

25. Procédé de diagnostic d'une inflammation chez un sujet mammifère, le procédé comprenant:
(a) l'incubation d'un échantillon de tissu provenant d'un sujet avec une composition contenant un anticorps chimérique anti-ICAM-1 marqué de manière détectable préparé par un procédé selon l'une quelconque des revendications 1 à 3, et
(b) la détection de tout anticorps chimérique lié à la cellule.
